# EUROPEAN PATENT APPLICATION

(11) **EP 3 663 315 A1**
(43) Date of publication of application: **10.06.2020**
(21) Application number: 20153125.8
(22) Date of filing: 28.10.2015
(51) Int. Cl.: C07K 16/10, C12N 15/00, A61K 39/145, C07K 16/00, A61K 39/12

(54) **DIRECT EXPRESSION OF ANTIBODIES**

(30) Priority: 29.10.2014 EP 14190943
(62) Divisional of application: 15797871.9
(71) Applicant: Novartis AG, 4056 Basel (CH); The General Hospital Corporation, Boston, MA 02114 (US)
(72) Inventor: Geall, Andrew, East Hanover, NJ 07936-1080 (US); Alter, Galit, Cambridge, MA 02139 (US); Suscovich, Todd, Cambridge, MA 02139 (US)
(74) Representative: V.O.

(57) **Abstract**

The present invention relates to methods exogenous nucleic acid molecules, such as RNA, that encode an antibody or antigen-binding fragment of an antibody, and optionally encode a cellular modulation factor and/or a potentiation factor. The cellular modulation factor is a factor that results in increased expression of the antibody and/or antigen-binding fragment. The potentiation factor is a factor that provides desired antibody effector or other properties. The exogenous nucleic acid molecules can be DNA or RNA, as mRNA (including self-replication RNA and non-self-replicating RNA). The invention also relates method for administering the exogenous nucleic acid molecules to a subject to achieve production of the encoded antibody or antigen-binding fragment in the subject to provide, for example, prophylactic or therapeutic passive immunity..

## Description

### FIELD OF THE INVENTION

This invention relates the use of exogenous nucleic acid molecules, such as RNA, for direct expression of antibody (or antigen-binding fragment of an antibody) to confer immunity.

### BACKGROUND OF THE INVENTION

Traditionally, antibody-mediated immunity against specific pathogens has been induced via vaccination, e.g., with an inactivated or attenuated form of the pathogen or with pathogen antigens. For many pathogens, vaccines that provide complete protection are not available, and it can take months or even years for some vaccines to achieve a sufficient degree of protective immunity.

An alternative way to provide antibody-mediated immunity to an animal is through passive immunity. Passive immunity is usually provided through a process in which protective antibodies or serum is transferred to a non-immune individual to confer immunity. Although these methods are effective and provide rapid protection, they are invasive and expensive.

More recently, the possibility of passive transfer of therapeutic antibodies using genes that encode antibodies, e.g., viral vectors that encode antibodies, has been investigated. See, *e.g.,* U.S. Patent Publication Number 2003/0147868; Skaricic et al., Virology 378:79-85 (2008); Rosenberg et al., Human Gene Ther., 23:451-59 (2012); Gupta et al., J Virol 75:4649-54 (2001); Subbarao et al., J Virol 78:3572-77 (2004); Osorio et al., Virology 302:9-20 (2002); Johnson et al., Nat Med 15:901-06 (2009); Balazs et al., Nature 481:81-84 (2012); each of which is incorporated herein by reference in its entirety. For example, the possibility of passive transfer of therapeutic monoclonal antibodies using adeno-associated virus (AAV) has been evaluated in studies using several pathogens. Gupta et al., J Virol 75:4649-54 (2001); Subbarao et al., J Virol 78:3572-77 (2004); Osorio et al., Virology 302:9-20 (2002); Johnson et al., Nat Med 15:901-06 (2009); Balazs et al., Nature 481:81-84 (2012). Certain viral vectors, particularly AAV-based vectors, can infect both dividing and non-dividing cells, persists as an extrachromosomal state without integrating into the host cell genome, confers moderately durable immunity, and can be administered by intramuscular injection. See, e.g., Vandenberghe LH, et al. Curr Gene Ther 7: 325-333 (2007). However, adeno-associated virus-mediated gene delivery has a number of limitations including: a) a small viral genome, which limits the quantity and type of genes that can be delivered; b) humoral immunity frequently develops against the vector, limiting its repeated use; and c) genes are delivered mainly to muscle cells, neurons, and hepatocytes, which do not naturally produce antibodies at sufficiently high titers or modify the antibodies in a manner analogous to B cells to potentiate their Fc-mediated effector functions. Similarly, non-viral delivery of DNA (*e.g.,* via electroporation) has consistently failed to produce sufficiently high titers of antibodies to confer immunity. The use of viral vectors is also hampered by the risk of accidental germline transmission, cancer, and/or antiviral immune responses which may prevent expression of the antibodies and/or cause unwanted side effects. See, *e.g.,* Bakker et al., Mol. Ther. 10:411-16 (2004) (incorporated by reference herein in its entirety).

A need exists for improved methods for achieving effective, robust, and rapid passive immunity. In particular a need exists for improved methods for direct expression of antibodies and antigen-binding fragments of antibodies in living subjects, such that sufficient levels of the antibodies and antigen-binding fragments of antibodies, with desired effector functions, are produced to confer robust and rapid immunity

### SUMMARY OF THE INVENTION

The present invention relates to methods exogenous nucleic acid molecules, such as RNAs, that encode an antibody or antigen-binding fragment of an antibody, and optionally encode a cellular modulation factor and/or a potentiation factor. The cellular modulation factor is a factor that results in increased expression of the antibody and/or antigen-binding fragment. The potentiation factor is a factor that provides desired antibody effector or other properties. The exogenous nucleic acid molecules can be DNA or RNA, such as mRNA (including self-replication RNA and non-self-replicating RNA). The invention also relates method for administering the exogenous nucleic acid molecules to a subject to achieve production of the encoded antibody or antigen-binding fragment in the subject to provide, for example, prophylactic or therapeutic passive immunity.

In one aspect, the invention provides a recombinant nucleic acid (e.g., RNA) molecule comprising: a polynucleotide sequence encoding an antibody, or antigen-binding fragment of an antibody, that is operably linked to one or more expression control elements, thereby in a suitable host cell resulting in the production of said antibody or antigen-binding fragment.

In certain embodiments, the nucleic acid molecule is DNA. The DNA may be a cDNA, or a DNA sequence that does not comprise an intron that can be found in the genomic sequences encoding immunoglobulins.

In certain embodiments, the nucleic acid molecule is RNA. The recombinant RNA molecule may be obtained by *in vitro* transcription.

To further enhance the therapeutic effect of the nucleic acid, in particular when introduced into a host cell that does not naturally produces antibodies, the nucleic acid molecule may further comprise: (i) an RNA sequence encoding a cellular modulation factor, (ii) an RNA sequence encoding a potentiation factor, or (iii) a combination thereof.

In some embodiments, the antibody, or antigen-binding fragment thereof, comprises a heavy chain or the V_{H} domain of the heavy chain, and a light chain or the V_{L} domain of the light chain. The coding sequence for the heavy chain or V_{H} domain, and the coding sequence for the light chain or V_{L} domain can be in a single open reading frame. The heavy chain or V_{H} domain, and the light chain or V_{L} domain may be covalently linked by a linker, which may be cleavable.

The recombinant nucleic acid molecules described herein may be delivered to a subject using a delivery system. Such delivery system include, e.g., a lipid nanoparticle, a polymer nanoparticle, a liposome, and an oil-in-water emulsion.

Also disclosed herein are methods of using the nucleic acid molecule (such as RNA) to confer immunity. The nucleic acid molecule (such as RNA) can be administered directly (e.g., by intramuscular injection) to a subject, wherein an antibody is expressed *in vivo.* Alternatively, cell-based therapy may be used. In a cell-based method, plasmablasts are transfected *in vitro* with the nucleic acid molecule (such as RNA) described herein. Then the plasmablasts are differentiated into mature antibody-secreting cells, which are then administered to a subject to confer immunity.

### DESCRIPTION OF THE DRAWINGS

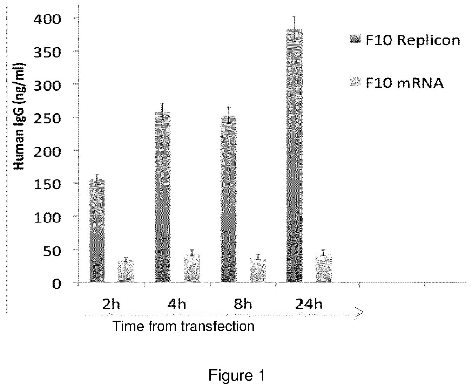

Figure 1 shows that Ab expression was detected in both from C2C12 cells transfected from replicons and C2C12 cells transfected with mRNA. Replicons resulted in higher Ab expression as compared to mRNA. Ab expression was quantified by IgG ELISA on C2C12 cells neon-transfected with an mRNA or replicon encoding the F10 influenza-specific antibody.

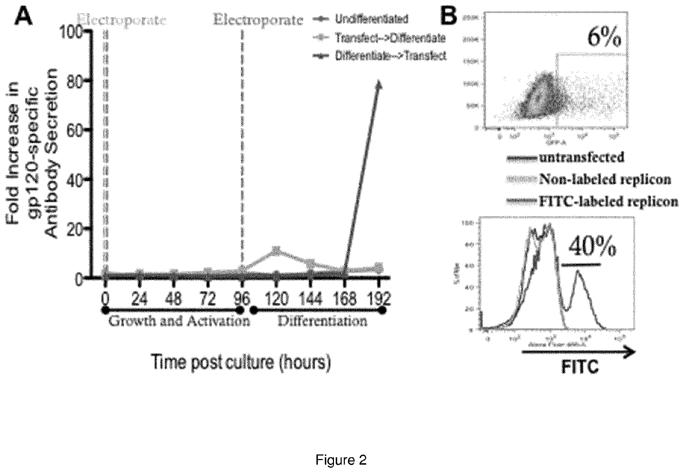

Figure 2 shows the results of replicon delivery to B cells. The line graph represents the level of a monoclonal antibody against HIV (B6) produced in culture supernatants from B cells transfected with a replicon at different stages of B cell differentiation (A), demonstrating the robust production of antibodies in mature transfected B cells which are then differentiation to ASCs. Similarly, the dot-plot and histogram (B) show that a small, but significant, fraction of peripheral blood plasmacells/plasmablasts can be transfected with replicon.

### DETAILED DESCRIPTION OF THE INVENTION

### 1. OVERVIEW

This disclosure relates to methods and materials for the passive transfer of antibodies and antigen-binding fragments thereof, for example to provide passive immunity in a mammalian subject (*e.g.,* to pathogens, tumors, *etc.* for therapeutic or protective purposes). In general, such methods involve administering an (one or more) exogenous nucleic acid molecule (such as an RNA molecule) that encodes an (one or more) antibody and/or antigen-binding fragment of an antibody to a subject, such that the antibody or antigen-binding fragment of the antibody can be directly expressed *in vivo* to confer immunity.

The inventors have demonstrated for the first time that an antibody and/or antigen-binding fragment of an antibody can be expressed *in situ* in cells of a living subject from exogenous RNA molecules that encode such antibodies (or antigen-binding fragments thereof). More significantly, the exogenous RNA molecule can be an mRNA (including self-replicating mRNA or non-self-replicating mRNA), instead of a viral vector. Difficulties of *in vivo* delivery of mRNA have been reported extensively, in particular the lack of stability of RNA molecules *in vivo* (e.g., degradation by serum nucleases) and host defense mechanisms against foreign RNA. Despite these difficulties, the inventors have demonstrated robust and reproducible production of antibodies *in vivo,* directly expressed from the exogenous RNA. Further, the nucleic acid molecule (e.g., RNA) of the invention does not need to be packaged into viral particles. Instead, non-viral delivery systems, such as lipid-nanoparticles ("LNPs") can be used.

As disclosed and exemplified herein, the exogenous nucleic acid molecule (such as an RNA molecule) can be delivered to a variety of host cells (such as muscle cells, liver cells, spleen cells etc.). B cells and plasmablasts are natural hosts for production and/or secretion of antibodies. When the nucleic acid molecule (such as RNA) is delivered to B cells and plasmablasts, efficient expression of antibodies is generally expected.

However, many cells (such as muscle cells, liver cells, spleen cells) are not natural hosts for production and/or secretion of antibodies. The inventors discovered that these host cells nonetheless are suitable for expressing antibodies from the exogenous nucleic acid (such as RNA). To further enhance the expression and/or potency of antibodies produced from non-natural host cells, the nucleic acid molecule (such as RNA) may further encode a cellular modulation factor and/or a potentiation factor. A cellular modulation factor can enhance the expression and/or secretion of antibodies from a non-natural host cell (such as a muscle cell). And a potentiation factor can enhance the potency of an antibody, e.g., through glycosylation, so that the antibody has a glycosylation pattern similar to antibodies produced by B cells and plasmablasts.

The inventors have surprisingly determined that the *in vivo* expression of an antibody and/or antigen-binding fragment of an antibody in a subject can be enhanced by co-expression of a cellular modulation factor, as described herein. The inventors have also determined that effector function and other properties of the expressed antibody or antigen-binding fragment (e.g., antibody dependent cell-mediated cytotoxicity, complement fixation, immunogenicity) can be tailored, as desired, by co-expression of a potentiation factor, such as a potentiation factor that can alter the glycosylation state of the expressed antibody and/or antigen-binding fragment of an antibody.

The RNA molecules used in the Examples disclosed herein also have several safety advantages. Since the RNA molecule cannot integrate into the host genome, there is no risk of malignancies or disrupting the expression of an essential gene. Further, because RNA molecules are inherently unstable, and cannot spread from one cell to another cell (like an RNA virus), expression of the antibodies is generally short-lived, avoiding uncontrolled long term expression of the antibodies. Therefore, preferably, for safety reasons, the nucleic acid molecules of the invention (such as RNA) do not integrate into the genome of the host cells.

Also disclosed herein are methods of using the nucleic acid molecule (such as RNA) to confer immunity. The nucleic acid molecule (such as RNA) can be administered directly (e.g., by intramuscular injection) to a subject, wherein an antibody is expressed *in vivo.* Alternatively, cell-based therapy may be used. In a cell-based method, plasmablasts are transfected *in vitro* with the nucleic acid molecule (such as RNA) described herein. Then the plasmablasts are differentiated into mature antibody-secreting cells, which are then administered to a subject to confer immunity.

### 2. EXOGENOUS NUCLEIC ACID MOLECULES

The exogenous nucleic acid molecules (such as RNAs) described herein encode (1) an antibody and/or antigen-binding fragment of an antibody, and optionally, one or both of (2) a cellular modulation factor which enhances expression of the antibody and/or antigen-binding fragment of an antibody, and/or (3) a potentiation factor. Each of these components is described in further detail below.

Exogenous nucleic acid molecules described herein can be DNA, RNA, or mixture or combination of DNA and RNA. Preferably, an exogenous nucleic acid molecule is an RNA molecule, more preferably an mRNA molecule, and most preferably a self-replicating RNA molecule. An exogenous nucleic acid molecule (such as an RNA molecule) can be a recombinant molecule.

An exogenous nucleic acid molecule (such as RNA) will, in general, include expression control elements for the expression of the encoded antibody and/or antigen-binding fragment of an antibody, and, if present, the optional cellular modulation factor and/or potentiation factor. For example, an exogenous DNA molecule will include a suitable promoter, poly A encoding sequence and other expression control elements that are operably linked to the coding sequence for the antibody and/or antigen-binding fragment of an antibody, cellular modulation factor, and/or potentiation factor. Accordingly, the DNA molecule can be transcribed in the target cell (as defined below) to produce an mRNA, that is then translated to produce the antibody and/or antigen-binding fragment of an antibody, cellular modulation factor, and/or potentiation factor.

When an exogenous nucleic acid molecule is an mRNA, it is preferably fully processed and includes a 5' cap structure and polyA tail. The mRNA can include any suitable 5' cap structure, such as the m⁷G cap, or a suitable cap analog, such as, GP₃G, m⁷GP₃G, m₃^{2,2,7}GP₃G, m₂^{7,3'}-OGP₃G, and the like. The mRNA can include a polyA tail. However, if desired, the mRNA can be partially processed, e.g., may contain one or more introns.

The exogenous nucleic acid molecules can be mono-cistronic and encode only an antibody and/or antigen-binding fragment of an antibody, or be multi-cistronic (contain two or more cistrons). Multi-cistronic nucleic acid molecules may contain two or more antibodies and/or antigen-binding fragments of an antibody. Alternatively or additionally, multi-cistronic nucleic acid molecules may contain one or more antibodies and/or antigen-binding fragments of an antibody, and also one or both of a cellular modulation factor and/or a potentiation factor.

When the exogenous nucleic acid molecule contains more than one cistron, each cistron will usually contain a coding sequence for an antibody and/or antigen-binding fragment of an antibody, cellular modulation factor, and/or potentiation factor operably linked to suitable expression control elements. For example, in a multi-cistronic RNA (*e.g.,* a bi-cistronic mRNA or self-replicating RNA) each cistron can contain a coding sequence for an antibody and/or antigen-binding fragment of an antibody, cellular modulation factor, and/or potentiation factor that is operably linked to a suitable expression control element, such as a subgenomic promoter, an internal ribosome entry site ("IRES," *e.g.,* EMCV, EV71, *etc.),* a viral 2A site, and the like. Any combination of the same or different expression control elements can be used to drive the expression of any of the cistrons contained in the exogenous nucleic acid molecule. To give but one example, in a multi-cistronic RNA, the antibody and/or antigen-binding fragment of an antibody may be operably linked to a subgenomic promoter, and a cellular modulation factor and/or potentiation factor may be operably linked to an IRES and/or a viral 2A site.

In general, exogenous nucleic acid molecules do not integrate into the genome of the target cell in which they have been introduced. Preferably, exogenous nucleic acid molecules, such as mRNA molecules and self-replicating RNA molecules, can be found exclusively in the cytoplasm of transfected target cells. Exogenous nucleic acid molecules may be found in and/or associated with one or more organelles contained within the target cell (e.g., endoplasmic reticulum).

If desired, an exogenous nucleic acid molecule can include one or more modified nucleotides. If desired, an exogenous nucleic acid molecule can contain chemical modifications in or on the sugar moiety of the nucleoside (e.g., ribose, deoxyribose, modified ribose, modified deoxyribose, six-membered sugar analog, or open-chain sugar analog), or the phosphate (e.g., phosphoramidate, phosphorothioate, and/or methylphosphonate linkages). An exogenous nucleic acid molecule may comprise modified nucleotides that contain a modification on or in the nitrogenous base, but do not contain modified sugar or phosphate moieties.

There are more than 96 naturally occurring nucleoside modifications found on mammalian RNA. See, e.g., Limbach et al., Nucleic Acids Research, 22(12):2183-2196 (1994). The preparation of nucleotides and modified nucleotides and nucleosides are well-known in the art, e.g. from US Patent Numbers 4,373,071, 4,458,066, 4,500,707, 4,668,777, 4,973,679, 5,047,524, 5,132,418, 5,153,319, 5,262,530, 5,700,642, each incorporated herein by reference in its entirety, and many modified nucleosides and modified nucleotides are commercially available. In certain embodiments, the exogenous nucleic acid molecule is mRNA that contains a modified nucleotide (in addition to the 5' cap), and about 1%, about 2%, about 3%, about 4%, about 5%, about 10%, about 15%, about 20%, or about 25% of the nucleotides are modified nucleotides.

When the exogenous nucleic acid molecule is an RNA molecule, it can be an mRNA or self-replicating RNA that contains one or more modified nucleotides.

Self-replicating RNA molecules, which are sometimes referred to as self-amplifying RNA molecules, are typically based on the genomic RNA of RNA viruses (e.g., positive-stranded RNA virus, including, but not limited to, alphavirus, picornavirus, flavivirus (e.g., Yellow Fever Virus and/or West Nile Virus), rubivirus, pestivirus, hepacivirus, calicivirus, and/or coronavirus), but lack the genes encoding one or more structural proteins. Self-replicating RNA molecules are capable of being translated to produce non-structural proteins of the RNA virus and heterologous proteins encoded by the self-replicating RNA. Any self-replicating RNA technology which is known in the art can be used in accordance with the present invention, including, but not limited to, technology described in co-pending U.S. patent application number 12/831,252, filed July 6, 2010, and published on December 8, 2011, as U.S. patent publication number 2011/0300205, the contents of which are incorporated herein by reference in its entirety. In general, a self-replicating RNA is about 5,000-25,000 nucleotide long (e.g., 5,000-20,000 nucleotide long, 5,000-19,000 nucleotide long, 5,000-18,000 nucleotide long, 5,000-17,000 nucleotide long, 5,000-16,000 nucleotide long, or 5,000-15,000 nucleotide long, etc.)

Self-replicating RNA molecules generally contains at least one or more genes selected from the group consisting of viral replicase, viral proteases, viral helicases and other nonstructural viral proteins, and also comprise 5'- and 3'-end cis-active replication sequences, and if desired, a heterologous sequence that encodes a desired amino acid sequence (*e.g.,* protein, antigen, *etc.*) or provides a non-coding, functional RNA (e.g., siRNA, miRNA, and/or other RNAi agent). Self-replicating RNAs often contain a subgenomic promoter that directs expression of the heterologous sequence. The heterologous sequence may be fused in frame to other coding regions in the self-replicating RNA and/or may be under the control of an internal ribosome entry site (IRES).

Whereas viral genomes (such as the genome of an alphavirus) encode structural virion proteins in addition to the non-structural replicase polyprotein, it is preferred that the self-replicating RNA molecules described herein do not encode viral structural proteins. Thus, a preferred self-replicating RNA can lead to the production of daughter RNAs in a host cell, but not to the production of RNA-containing virions.

Exogenous nucleic acid molecules can be of any suitable size, for example, shorter than about 100 nucleotides, about 200 nucleotides, about 500 nucleotides, about 1000 nucleotides, about 2000 nucleotides, about 3000 nucleotides, about 4000 nucleotides, about 5000 nucleotides, about 10,000 nucleotides, about 15,000 nucleotides, about 20,000 nucleotides, or about 25,000 nucleotides in length. Exogenous nucleic molecules can be longer than 25,000 nucleotides in length. Exogenous nucleic acid molecules can be between about 100 and about 1000 nucleotides, between about 500 and about 1000 nucleotides, between about 500 and about 2000 nucleotides, between about 500 and about 5000 nucleotides, between about 1000 and about 10,000 nucleotides, between about 1000 and about 15,000 nucleotides, between about 1000 and about 20,000 nucleotides, or between about 1000 and about 25,000 nucleotides in length.

Exogenous nucleic acid molecules can be produced using any suitable method. For example, DNA can be produced synthetically or using conventional recombinant techniques. Similarly, RNA can be produced by *in vitro* transcription, produced by chemical synthesis, and/or purified from suitable cells (e.g., recombinant cells, cells obtained from a living or deceased subject, commercially-available cell stocks, *etc.).* Suitable methods are well-known in the art.

In some aspects, the exogenous nucleic acid molecule is not a nucleic acid that can integrate into the genome of a target cell, is not a viral vector and/or is not a DNA molecule.

### Antibodies and/or Antigen-Binding Antibody Fragments

Exogenous nucleic acid molecules contain a nucleotide sequence that encodes an antibody and/or antigen-binding fragment of an antibody, such as an antibody that provides protective immunity or therapeutic activity. The encoded antibody or antigen-binding fragment can be any antibody or antigen-binding fragment that has desired binding specificity. The encoded antibody or antigen-binding fragment can be of any desired isotype, for example IgA (*e.g.,* IgA1, IgA2), IgD, IgE, IgG (*e.g.,* IgG1, IgG2, IgG3, IgG4), or IgM. Light chains, if present, may be of any subtype, including lambda, kappa, and/or iota subtypes. Heavy chains, if present, may be of any subtype, including alpha, delta, epsilon, gamma, and/or mu subtypes.

An antigen-binding fragment of an antibody can be any portion of an antibody that has binding specificity for antigen. An antigen-binding fragment of an antibody can be, for example, a Fab, F(ab)'₂, Fv region, scFv region, complementarity determining region ("CDR"), dAbs, functional fragments thereof, and/or combinations thereof. An antigen-binding fragment can also be a single heavy chain or single light chain that binds antigen.

The encoded antibody or antigen-binding fragment can include portions that are derived from different antibodies and recombined. For example, the exogenous nucleic acid molecule can encode a CDR grafted antibody (e.g., humanized antibody) or chimeric antibody, or an antigen binding portion of such an antibody. If desired the antibody or antigen-binding fragment can have binding specificity for two or more antigens. For example, the encoded antibody can be a bi-specific antibody. A bispecific antibody can contain one variable region (e.g., V_{H} plus V_{L}) that has binding specificity for a first antigen, and a second variable region that has binding specificity for a second different antigen. Generally, it is preferred that the antibody or antigen-binding fragment is from the same species as the intended recipient of the exogenous nucleic acid molecule. For example, when the exogenous nucleic acid molecule is intended for administration to a human, it is preferred that the encoded antibody or antigen-binding fragment is a human antibody or antigen-binding fragment thereof.

Antibodies and antigen-binding fragments that are encoded by the exogenous nucleic acid molecules have binding specificity for one or more desired target antigens. The target antigen(s) can be a protein, nucleic acid, carbohydrate, lipid, glycoprotein, proteoglycan, small organic molecule, virus, cell and the like. Target antigens may be associated with a pathogen, such as a virus, bacterium, fungus, parasite, plant, and/or other pathogenic and/or poisonous organism. Pathogen-associated target antigens may be found on the surface of, contained within, or secreted from a pathogen of interest. Target antigens can be molecules that are endogenous to the intended recipient of the exogenous nucleic acid molecule. For example, the antigen can be an endogenous molecule that is associated with pathology, such as a molecule that is altered (e.g., by mutation, post-translational processing, etc.) or differentially expressed (e.g., expressed at higher or lower levels) on diseased cells and healthy cells. In one example, the endogenous target antigen is associated with a cell proliferation disorder, such as cancer. Endogenous target antigens can be found on the surface of, contained within, or secreted from a cell of interest.

If desired, an exogenous nucleic acid molecule can encode an antibody or antigen-binding fragment that includes certain amino acids in the constant and/or variable regions to provide desired processing and/or effector functions. For example, the amino acid sequence of an antibody constant region can be altered to add or remove sites for N-linked glycosylation, can include an amino acid sequence that reduced complement fixation, and/or that alters binding to Fc receptors. An exogenous nucleic acid molecule can encode an antibody or antigen-binding fragment that has one, more than one, or all of the amino acid mutations set forth in Table 1.

**Table 1: Exemplary Antibody Mutations**

| |
|---|
| A330L |
| E345R |
| E430G |
| G236A |
| H268F |
| I332E |
| L234A |
| L235A |
| M428L |
| N434S |
| S239D |
| S267E |
| S324T |
| S440Y |

In certain embodiments, the antibody, or antigen-binding fragment thereof, comprises a heavy chain or the V_{H} domain of the heavy chain, and a light chain or the V_{L} domain of the light chain. In certain embodiments, the coding sequence for said heavy chain or V_{H} domain, and the coding sequence for said light chain or V_{L} domain are in a single open reading frame.

In certain embodiments, the exogenous nucleic acid molecule comprises one or more expression control elements that are located between the coding sequence for said heavy chain or V_{H} domain, and the coding sequence for said light chain or V_{L} domain. For example, an IRES can be inserted between the coding sequence for the heavy chain, and the coding sequence for the light chain. Although only one RNA transcript is introduced into a cell, the heavy chain and light chain coding sequences each has its own a ribosome entry site, such that two chains can be translated separately. Such expression control element can be a subgenomic promoter, an IRES, a viral 2A site.

In certain embodiments, the heavy chain or V_{H} domain, and said light chain or V_{L} domain are covalently linked by a linker. The linker may be cleavable. The linker can be 3-30 amino acid long (e.g., 10-20 amino acid long). A linker region can promote appropriate interactions between V_{H} domain and V_{L} domain.

In some preferred aspects, an exogenous nucleic acid molecule encodes an antibody and/or antigen-binding fragment of an antibody that recognizes and binds specifically to a pathogen-associated antigen. For example, viral antigens that can be recognized and specifically bound by an antibody and/or antigen-binding fragment of an antibody include, but are not limited to, antigens, such as proteins and peptides, from Orthomyxoviruses, such as Influenza A, B and C; Paramyxoviridae viruses, such as Pneumoviruses, Paramyxoviruses (PIV), Metapneumovirus and Morbilliviruses (e.g., measles); Pneumoviruses, such as Respiratory syncytial virus (RSV), Bovine respiratory syncytial virus, Pneumonia virus of mice, and Turkey rhinotracheitis virus; Paramyxoviruses, such as Parainfluenza virus types 1 - 4 (PIV), Mumps, Sendai viruses, Simian virus 5, Bovine parainfluenza virus, Nipahvirus, Henipavirus and Newcastle disease virus; Poxviridae, such as *Variola vera,* including but not limited to, *Variola major* and *Variola minor*; Metapneumoviruses, such as human metapneumovirus (hMPV) and avian metapneumoviruses (aMPV); Morbilliviruses, such as Measles; Picornaviruses, such as Enteroviruses, Rhinoviruses, Heparnavirus, Parechovirus, Cardioviruses and Aphthoviruses; Enteroviruseses, such as Poliovirus types 1, 2 or 3, Coxsackie A virus types 1 to 22 and 24, Coxsackie B virus types 1 to 6, Echovirus (ECHO) virus) types 1 to 9, 11 to 27 and 29 to 34 and Enterovirus 68 to 71, Bunyaviruses, such as California encephalitis virus; a Phlebovirus, such as Rift Valley Fever virus; a Nairovirus, such as *Crimean-Congo hemorrhagic fever* virus; Heparnaviruses, such as, Hepatitis A virus (HAV); Togaviruses, such as a Rubivirus, an Alphavirus, or an Arterivirus; Flaviviruses, such as Tick-borne encephalitis (TBE) virus, Dengue (types 1, 2, 3 or 4) virus, Yellow Fever virus, Japanese encephalitis virus, Kyasanur Forest Virus, West Nile encephalitis virus, St. Louis encephalitis virus, Russian spring-summer encephalitis virus, Powassan encephalitis virus; Pestiviruses, such as Bovine viral diarrhea (BVDV), Classical swine fever (CSFV) or Border disease (BDV); Hepadnaviruses, such as Hepatitis B virus, Hepatitis C virus; Rhabdoviruses, such as a Lyssavirus (Rabies virus) and Vesiculovirus (VSV), Caliciviridae, such as Norwalk virus, and Norwalk-like Viruses, such as Hawaii Virus and Snow Mountain Virus; Coronaviruses, such as SARS, Human respiratory coronavirus, Avian infectious bronchitis (IBV), Mouse hepatitis virus (MHV), and Porcine transmissible gastroenteritis virus (TGEV); Retroviruses such as an Oncovirus, a Lentivirus or a Spumavirus; Reoviruses, as an Orthoreovirus, a Rotavirus, an Orbivirus, or a Coltivirus; Parvoviruses, such as Parvovirus B19; Delta hepatitis virus (HDV); Hepatitis E virus (HEV); Human Herpesviruses, such as, by way Herpes Simplex Viruses (HSV), Varicella-zoster virus (VZV), Epstein-Barr virus (EBV), Cytomegalovirus (CMV), Human Herpesvirus 6 (HHV6), Human Herpesvirus 7 (HHV7), and Human Herpesvirus 8 (HHV8); Papovaviruses, such as Papillomaviruses and Polyomaviruses, Adenoviruess and Arenaviruses; Ebola virus; Human Immunodeficiency Virus 1 (HIV1) and Human Immunodeficiency Virus 2 (HIV2); and/or any combination of the foregoing.

Bacterial antigens that can be recognized and specifically bound by an antibody and/or antigen-binding fragment of an antibody include, but are not limited to, antigens, such as proteins and peptides, from *Neisseria meningitides, Streptococcus pneumoniae, Streptococcus pyogenes, Moraxella catarrhalis, Bordetella pertussis, Burkholderia sp.* (*e.g., Burkholderia mallei, Burkholderia pseudomallei and Burkholderia cepacia), Staphylococcus aureus, Haemophilus influenzae, Clostridium tetani* (Tetanus), *Clostridium perfringens, Clostridium botulinums, Cornynebacterium diphtheriae* (Diphtheria), *Pseudomonas aeruginosa, Legionella pneumophila, Coxiella burnetii, Brucella* sp. (*e.g., B. abortus, B. canis, B. melitensis, B. neotomae, B. ovis, B. suis and B. pinnipediae*), *Francisella sp.* (*e.g., F. novicida, F. philomiragia* and *F. tularensis*), *Streptococcus agalactiae, Neiserria gonorrhoeae, Chlamydia trachomatis, Treponema pallidum* (Syphilis), *Haemophilus ducreyi, Enterococcus faecalis, Enterococcus faecium, Helicobacter pylori, Staphylococcus saprophyticus, Yersinia enterocolitica, E. coli, Bacillus anthracis* (anthrax), *Yersinia pestis* (plague), *Mycobacterium tuberculosis, Rickettsia, Listeria, Chlamydia pneumoniae, Vibrio cholerae, Salmonella typhi* (typhoid fever), *Borrelia burgdorfer, Porphyromonas sp, Klebsiella sp., Clostridium difficile,* and/or any combination of the foregoing.

Fungal antigens that can be recognized and specifically bound by an antibody and/or antigen-binding fragment of an antibody include, but are not limited to, antigens, such as proteins and peptides, from Dermatophytres, including: *Epidermophyton floccusum, Microsporum audouini, Microsporum canis, Microsporum distortum, Microsporum equinum, Microsporum gypsum, Microsporum nanum, Trichophyton concentricum, Trichophyton equinum, Trichophyton gallinae, Trichophyton gypseum, Trichophyton megnini, Trichophyton mentagrophytes, Trichophyton quinckeanum, Trichophyton rubrum, Trichophyton schoenleini, Trichophyton tonsurans, Trichophyton verrucosum, T. verrucosum* var. album, var. discoides, var. ochraceum, *Trichophyton violaceum,* and/or *Trichophyton faviforme;* or from *Aspergillus fumigatus, Aspergillus flavus, Aspergillus niger, Aspergillus nidulans, Aspergillus terreus, Aspergillus sydowi, Aspergillus flavatus, Aspergillus glaucus, Blastoschizomyces capitatus, Candida albicans, Candida enolase, Candida tropicalis, Candida glabrata, Candida krusei, Candida parapsilosis, Candida stellatoidea, Candida kusei, Candida parakwsei, Candida lusitaniae, Candida pseudotropicalis, Candida guilliermondi, Cladosporium carrionii, Coccidioides immitis, Blastomyces dermatidis, Cryptococcus neoformans, Geotrichum clavatum, Histoplasma capsulatum, Klebsiella pneumoniae, Microsporidia, Encephalitozoon* spp., *Septata intestinalis* and *Enterocytozoon bieneusi*; the less common are *Brachiola* spp, *Microsporidium* spp., *Nosema* spp., *Pleistophora* spp., *Trachipleistophora* spp., *Vittaforma* spp *Paracoccidioides brasiliensis, Pneumocystis carinii, Pythiumn insidiosum, Pityrosporum ovale, Sacharomyces cerevisae, Saccharomyces boulardii, Saccharomyces pombe, Scedosporium apiosperum, Sporothrix schenckii, Trichosporon beigelii, Toxoplasma gondii, Penicillium marneffei, Malassezia spp., Fonsecaea spp., Wangiella spp., Sporothrix spp., Basidiobolus spp., Conidiobolus spp., Rhizopus spp, Mucor spp, Absidia spp, Mortierella spp, Cunninghamella spp, Saksenaea spp., Alternaria spp, Curvularia spp, Helminthosporium spp, Fusarium spp, Aspergillus spp, Penicillium spp, Monolinia spp, Rhizoctonia spp, Paecilomyces spp, Pithomyces spp, Cladosporium spp.,* and/or any combination of the foregoing.

Protozoan antigens that can be recognized and specifically bound by an antibody and/or antigen-binding fragment of an antibody include, but are not limited to, antigens, such as proteins and peptides, from malaria parasite (e.g., *Plasmodium vivax, Plasmodium ovale, Plasmodium malariae*), *Entamoeba histolytica, Giardia lambli, Cryptosporidium parvum, Cyclospora cayatanensis, Toxoplasma, Naegleria fowleri, Trichomonas vaginalis, Balantidium coli, Leshmania spp., Cystiosospora* and/or any combination of the foregoing.

Plant antigens that can be recognized and specifically bound by an antibody and/or antigen-binding fragment of an antibody include, but are not limited to, antigens, such as proteins and peptides, from *Ricinus communis,* which produced the toxin ricin.

Small molecule antigens that can be recognized and specifically bound by an antibody and/or antigen-binding fragment of an antibody include, but are not limited to, antigens, such as drug substances (e.g., cocaine; nicotine, amphetamine, methamphetamine, morphine, hydrocodone, diacetylmorphine, lysergic acid diethylamide, phencyclidine); hormones (*e.g.,* estrogen, testosterone, and derivatives thereof); neurotransmitters (*e.g*., serotonin, NMDA, GABA); amino acids; nucleic acids; and so forth.

In other preferred aspects, an exogenous nucleic acid molecule encodes an antibody and/or antigen-binding fragment of an antibody that binds specifically to an endogenous antigen, such as an antigen associated with cancer or other proliferative disease. For example, the antibody or antigen-binding fragment can bind a tumor antigen, which include, but are not limited to,
(a) cancer-testis antigens such as NY-ESO-1, SSX2, SCP1 as well as RAGE, BAGE, GAGE and MAGE family polypeptides, for example, GAGE-1, GAGE-2, MAGE-1, MAGE-2, MAGE-3, MAGE-4, MAGE-5, MAGE-6, and MAGE-12 (which can be used, for example, to address melanoma, lung, head and neck, NSCLC, breast, gastrointestinal, and bladder tumors);
(b) mutated antigens, for example, p53 (associated with various solid tumors, *e.g.,* colorectal, lung, head and neck cancer), p21/Ras (associated with, *e.g.,* melanoma, pancreatic cancer and colorectal cancer), CDK4 (associated with, *e.g.,* melanoma), MUM1 (associated with, e.g., melanoma), caspase-8 (associated with, *e.g.,* head and neck cancer), CIA 0205 (associated with, *e.g.,* bladder cancer), HLA-A2-R1701, beta catenin (associated with, *e.g.,* melanoma), TCR (associated with, *e.g.,* T-cell non-Hodgkins lymphoma), BCR-abl (associated with, *e.g.,* chronic myelogenous leukemia), triosephosphate isomerase, KIA 0205, CDC-27, and LDLR-FUT;
(c) over-expressed antigens, for example, Galectin 4 (associated with, *e.g.,* colorectal cancer), Galectin 9 (associated with, *e.g.,* Hodgkin's disease), proteinase 3 (associated with, *e.g.,* chronic myelogenous leukemia), WT 1 (associated with, *e.g.,* various leukemias), carbonic anhydrase (associated with, *e.g.,* renal cancer), aldolase A (associated with, *e.g.,* lung cancer), PRAME (associated with, *e.g.,* melanoma), HER-2/neu (associated with, *e.g.,* breast, colon, lung and ovarian cancer), alpha-fetoprotein (associated with, *e.g.,* hepatoma), KSA (associated with, *e.g.,* colorectal cancer), gastrin (associated with, *e.g.,* pancreatic and gastric cancer), telomerase catalytic protein, MUC-1 (associated with, *e.g.,* breast and ovarian cancer), G-250 (associated with, *e.g.,* renal cell carcinoma), p53 (associated with, *e.g.,* breast, colon cancer), and carcinoembryonic antigen (associated with, *e.g.,* breast cancer, lung cancer, and cancers of the gastrointestinal tract such as colorectal cancer);
(d) shared antigens, for example, melanoma-melanocyte differentiation antigens such as MART-1/Melan A, gp100, MC1R, melanocyte-stimulating hormone receptor, tyrosinase, tyrosinase related protein-1/TRP1 and tyrosinase related protein-2/TRP2 (associated with, *e.g.,* melanoma);
(e) prostate associated antigens such as PAP, PSA, PSMA, PSH-P1, PSM-P1, PSM-P2, associated with *e.g.,* prostate cancer; and
(f) immunoglobulin idiotypes (associated with myeloma and B cell lymphomas, for example); fragments thereof; and/or any combination of the foregoing.

Tumor antigens that can be recognized and specifically bound by an antibody and/or antigen-binding fragment of an antibody also included, but are not limited to, endogenous and pathogen antigens, such as: p15, Hom/Mel-40, H-Ras, E2A-PRL, H4-RET, IGH-IGK, MYL-RAR, Epstein Barr virus antigens, EBNA, human papillomavirus (HPV) antigens, including E6 and E7, hepatitis B and C virus antigens, human T-cell lymphotropic virus antigens, TSP-180, p185erbB2, p180erbB-3, c-met, mn-23H1, TAG-72-4, CA 19-9, CA 72-4, CAM 17.1, NuMa, K-ras, p16, TAGE, PSCA, CT7, 43-9F, 5T4, 791 Tgp72, beta-HCG, BCA225, BTAA, CA 125, CA 15-3 (CA 27.29\BCAA), CA 195, CA 242, CA-50, CAM43, CD68\KP1, CO-029, FGF-5, Ga733 (EpCAM), HTgp-175, M344, MA-50, MG7-Ag, MOV18, NB/70K, NY-CO-1, RCAS1, SDCCAG16, TA-90 (Mac-2 binding protein\cyclophilin C-associated protein), TAAL6, TAG72, TLP, TPS, fragments thereof, and/or any combination of the foregoing.

An exogenous nucleic acid molecule may encode an antibody and/or antigen-binding fragment of an antibody that binds specifically to an endogenous antigen, such as a cytokine (e.g., interleukins, lymphokines, monokines, interferons, colony stimulating factors, and/or chemokines). Exemplary cytokines and/or cytokine-associated proteins include, but are not limited to, Adipokine, Albinterferon, CCL1, CCL11, CCL12, CCL13, CCL14, CCL15, CCL16, CCL17, CCL18, CCL19, CCL2, CCL20, CCL21, CCL22, CCL23, CCL24, CCL25, CCL26, CCL27, CCL28, CCL3, CCL5, CCL6, CCL7, CCL8, CCL9, CX3CL1, CX3CR1, CXCL1, CXCL10, CXCL11, CXCL13, CXCL14, CXCL15, CXCL16, CXCL17, CXCL2, CXCL3, CXCL5, CXCL6, CXCL7, CXCL9, Erythropoietin, Gc-MAF, Granulocyte colony-stimulating factor, Granulocyte macrophage colony-stimulating factor, Hepatocyte growth factor, IL-13, IL-19, IL-20, IL-22, IL-24, IL-26, IL17A, IL17B, IL17C, IL17D, IL17E (IL25), IL17F, ILIA, IL1B, Inflammasome, Interferome, Interferon, Interferon alpha, Interferon beta 1a, Interferon beta 1b, Interferon gamma, Interferon type I, Interferon type II, Interferon type III, Interleukin 1 family, Interleukin 1 receptor antagonist, Interleukin 10, Interleukin 12, Interleukin 12 subunit beta, Interleukin 13, Interleukin 16, Interleukin 2, Interleukin 23, Interleukin 23 subunit alpha, Interleukin 34, Interleukin 35, Interleukin 6, Interleukin 7, Interleukin 8, Interleukin-36, Leukemia inhibitory factor, Leukocyte-promoting factor, Lymphotoxin, Lymphotoxin alpha, Lymphotoxin beta, Macrophage colony-stimulating factor, Macrophage inflammatory protein, Macrophage-activating factor, Myonectin, Oncostatin M, Platelet factor 4, Promegapoietin, RANKL, Stromal cell-derived factor 1, Tumor necrosis factor alpha, XCL1, XCL2, XCR1, fragments thereof, and/or combinations thereof.

An exogenous nucleic acid molecule may encode an antibody and/or antigen-binding fragment of an antibody that binds specifically to one or more of the following antigens: CD3; CD11a; CD20; CD30; CD33; CD41, CD52; Integrin; GPIIb/IIIa (integrin IIb 3); Integrin 4; a4b7 integrin; IL-1b; IL-2R; IL-6; IL-6R; IL-12; IL-17a; IL-23; TNF-α; TNF-β; HER2; IgE; EGFR; VEGF; C5; EpCAM; RANK-L; BAFF; CTLA-4; HER2; PD-1; VEGFR2; GD2; PD-1; B7-H3; beta-amyloid; NARP-1; CEA; mesothelin; HLA-DR; L-selectin; RTN4; Rhesus factor; CD25; phosphatidylserine; CD22; CD125; CDA-related antigen; VEGF-A; fibrin II; ACVR2B; CD44v6; SOST; mucin CanAg; MUC1; VWF; MCP-1; Lewis-Y antigen; CD4; IGF-1 receptor; TRAIL-R2; TFPI; insulin-like growth factor receptor; DLL4; DR5; HER3; IL-4; ILGF2; GD3 ganglioside; LFA-1; CD11a; SLAMF7; TWEAK receptor; ICAM-1 (CD54); IL-9; 5AC; ITGB2 (CD18); PCSK9; CD15; folate receptor; HNGF; HGF; TYRP1; CD3 epsilon; nerve growth factor; CD80; CD147 (basigin); GPNMB; CD23 (IgE receptor); CLDN18.2; cardiac myosin; SDC1; CD51; CD152; CD6; PDCD1; NCA-90 (granulocyte antigen); IGHE; KIR2D; CD23; ch4D5; IL-5; CD74; CCR4; C242 antigen; RON; angiopoietin 2; IgG4; PDGF-R-alpha; human scatter factor receptor kinase; TEM1; oxLDL; CD37; OX-40; NOGO-A; EGFL7; LTA; CD79B; CCR5; N-glycolylneuraminic acid; fibronectin extra domain B; RHD; sclerostin; CD154 (CD40L); CD200; FAP; LOXL2; CD2; tenascin C; CD221; CD28; CD140a; MS4A1; 4-1BB; frizzled receptor; AOC3 (CAP-1); ITGA2; CD70; tumor antigen CTAA16.88; fragments thereof; and/or any combination of the foregoing.

The methods described herein can use, and target cells can contain, an exogenous nucleic acid molecule that encodes more than one antibody and/or antigen-binding fragment of an antibody (*e.g.*, a multi-cistronic mRNA) or two or more different exogenous nucleic acid molecules that provide two or more different antibodies and/or antigen-binding fragments of antibodies.

### Cellular Modulation Factors

Exogenous nucleic acid molecules can optionally contain a nucleotide sequence encoding a cellular modulation factor. As described herein, a cellular modulation factor increases expression of the antibody and/or antigen-binding fragment of an antibody encoded by the exogenous nucleic acid molecule, relative to the expression obtained in the absence of the cellular modulation factor. The cellular modification factor, can be, for example a protein or peptide that cause a cell to differentiate, causes a cell to terminally differentiate, or causing a change in the physiology of a cell (e.g., by expanding the endoplasmic reticulum and/or Golgi apparatus). For a cell that is not a natural host for antibody production, a cellular modulation factor may be used.

In general, cellular modulation factors may include cytokines, growth factors, transcription factors, chaperone proteins, translocation proteins, processing proteins and transporter proteins.

Cytokines and growth factors that can be used as cellular modulation factors include, but are not limited to, those that can promote the maturation of B cells into plasma cells (*i.e*., plasmablasts). Such cytokines and growth factors include, but are not limited to Adipokine, Albinterferon, CCL1, CCL11, CCL12, CCL13, CCL14, CCL15, CCL16, CCL17, CCL18, CCL19, CCL2, CCL20, CCL21, CCL22, CCL23, CCL24, CCL25, CCL26, CCL27, CCL28, CCL3, CCL5, CCL6, CCL7, CCL8, CCL9, CX3CL1, CX3CR1, CXCL1, CXCL10, CXCL11, CXCL13, CXCL14, CXCL15, CXCL16, CXCL17, CXCL2, CXCL3, CXCL5, CXCL6, CXCL7, CXCL9, Erythropoietin, Gc-MAF, Granulocyte colony-stimulating factor, Granulocyte macrophage colony-stimulating factor, Hepatocyte growth factor, IL-19, IL-20, IL-22, IL-24, IL-26, IL17A, IL17B, IL17C, IL17D, IL17E (IL25), IL17F, ILIA, IL1B, Inflammasome, Interferome, Interferon, Interferon alpha, Interferon beta 1a, Interferon beta 1b, Interferon gamma, Interferon type I, Interferon type II, Interferon type III, Interleukin 1 family, Interleukin 1 receptor antagonist, Interleukin 10, Interleukin 12, Interleukin 12 subunit beta, Interleukin 13, Interleukin 16, Interleukin 2, Interleukin 23, Interleukin 23 subunit alpha, Interleukin 34, Interleukin 35, Interleukin 6, Interleukin 7, Interleukin 8, Interleukin-36, Leukemia inhibitory factor, Leukocyte-promoting factor, Lymphotoxin, Lymphotoxin alpha, Lymphotoxin beta, Macrophage colony-stimulating factor, Macrophage inflammatory protein, Macrophage-activating factor, Myonectin, Oncostatin M, Platelet factor 4, Promegapoietin, RANKL, Stromal cell-derived factor 1, Tumor necrosis factor alpha, XCL1, XCL2, XCR1, fragments thereof, and/or combinations thereof.

Transcription factors that can be used as cellular modulation factors include, but are not limited to, ATF6, DNAJC3, PRDM1, XBP1(S), XBP1(U), and combinations thereof.

Chaperone proteins that can be used as cellular modulation factors include, but are not limited to, BAG1, BAG2, BAG3, BAG4, BAG5, BAG6, CALR, CANX, DNAJA1, DNAJA2, DNAJB1, DNAJB11, DNAJB1P1, DNAJB4, DNAJB9, DNAJC1, DNAJC10, ERO1L, ERO1LB, ERP44, HSP90B1, HSPA1A, HSPH1, HYOU1, MZB1, PDIA2, PDIA3, PDIA5, PDIA6, PPIB, SIL1, and combinations thereof.

Translocation proteins that can be used as cellular modulation factors include, but are not limited to, CKAP4, GCSI, RRBP1, SEC11C, SEC61A1, SEC61A2, SEC61B, SEC61G, SEC62, SEC63, SPCS2, SPCS3, SRP14, SRP19, SRP54, SRP68, SRP9, SRPR, SRPRB, SSR1, SSR2, SSR3, SSR4, SPCS1, and combinations thereof.

Post-translational processing proteins that can be used as cellular modulation factors include, but are not limited to, ALG1, ALG2, ALG3, ALG5, ALG6, DPAGT1, ALG8, ALG9, ALG10, ALG10B, ALG11, ALG12, ALG13, ALG14, MOGS, GANAB, MAN1, MGAT1, MAN2, MGAT2, DDOST, GLU1, GLU2, LMAN1, LMAN1L, LMAN2, OSTC, PREB, RPN1, RPN2, SARA1A, SERP1, STT3A, STT3B, UGGT1, UGGT2, and combination thereof.

Intracellular transport proteins that can be used as cellular modulation factors include, but are not limited to, BET1, GOSR1, GOSR2, SCFD1, SCFD2, SEC13, BNIP1, SEC22, SEC23A, SEC23B, SEC24A, SEC24C, SEC24D, SEC31A, SNAP23, SNAP29, STX1A, STX1B, STX2, STX3, STX4, STX5, STX6, STX7, STX8, STX10, STX11, STX12, STX13, STX14, STX16, STX17, STX18, STX19, STXBP1, STXBP2, STXBP3, STXBP4, STXBP5, STXBP6, USE1, VAMP4, YKT6, and combinations thereof.

In certain aspects, the exogenous nucleic acid molecule encodes an antibody or antigen-binding fragment thereof and a (one or more) cellular modulation factor that can cause expansion of the endoplasmic reticulum and or Golgi apparatus. Preferably, the cellular modulation factor causes expansion of the endoplasmic reticulum and or Golgi apparatus in muscle cells. Such cellular modulation factors include, for example, the transcription factors, chaperones, translocation proteins, processing proteins and transport proteins disclosed herein.

### Potentiating Factors

Exogenous nucleic acid molecules may optionally contain a nucleotide sequence encoding a potentiation factor. As described herein, a potentiation factor results can alter effector function and other properties of the expressed antibody or antigen-binding fragment (e.g., antibody dependent cell-mediated cytotoxicity, antibody dependent-cellular phagocytosis, complement fixation, immunogenicity). In preferred aspects, the potentiation alters the glycosylation of the antibody and/or antigen-binding fragment, relative to the glycosylation that is obtained in the absence of the potentiation factor. For example, potentiation factors may function by increasing or decreasing the expression and/or function of one or more glycosylation enzymes. Altered expression and/or function of a glycosylation enzyme may result in a cell that preferentially and/or exclusively produces a specific glycoform of an antibody and/or antigen-binding fragments of an antibody. In some instances, different glycoforms of an antibody and/or antigen-binding fragment of an antibody may display differences in activity (e.g., strength and/or specificity of antigen binding) and/or stability, *etc.*

For example, in its natural host (e.g., B cell), an antibody is typically afucosylated at its Fc region, whereas when produced in other types of cells, the Fc region is often fucosylated. For many antibodies, fucosylation of the Fc region plays an important role in antibody dependent dell mediated cytotoxicity (ADCC) and complement dependent cytotoxicity (CDC). Therefore, a potentiation factor may be introduced to modify the fucosylation pathway.

Potentiation factors may be proteins or peptides that bind to and either increase or decrease the activity of enzymes involved in glycosylation. Potentiation factors may be functional, non-coding RNAs (*e.g.,* siRNA, miRNA, antisense RNA, *etc*.) that decrease expression of enzymes involved in glycosylation.

Exemplary enzymes involved in glycosylation whose levels and/or activity may be modulated by potentiation factors include, but are not limited to, glycosyltransferase, fucosyltransferase (*e.g.*, FUT8), oligosaccharyltransferase, mannosidase I, Glc*N*Ac transferase, galactosyltransferase, sialyltransferase, UDP-*N*-acetyl-D-galactosamine:polypeptide *N*-acetylgalactosaminyltransferase, GDP-fucose protein O-fucosyltransferase 1, GDP-fucose protein O-fucosyltransferase 2, protein:*O*-glucosyltransferase (Poglut), *O*-Glc*N*Ac transferase (OGT), *O*-Glc*N*Acase (OGA), Fut8, Mgat3, Mgat5, Mgat5b, Mgat4c, Mgat2, Mgat4b, Mgat4a, GnT1IP, B4Galt1, B4Galt2, B4Galt3, St6Gal1, St6Gal2, Mgat1, Mogs, Ganab, Man1a, Man1a2, Man1b1, Man1c1, Man2a2, Man2a1, Alg1, Alg2, Alg3, Alg5, Alg6, Dpagt1, Alg8, Alg9, Alg10b, Alg11, Alg12, Alg13, Alg14, Glt28D2, Gmds, Tsta3, Fpgt, Fuk, Gale, Galt, Galk1, Galk2, Uap1, Uap1l1, Pgm3, Cmas, Gne, Nans, Nanp, Fuct1, AGA, FUCA1, FUCA2, ENGASE, MANBA, MAN2B2, MAN2C1, MAN2B1, HEXA, HEXB, GLB1, NEU1, NEU2, NEU3, and/or combinations thereof.

In some aspects, the potentiation factor inhibits fucosyltransferase levels and/or activity. More preferably, FUT8 levels and/or activity may be inhibited by potentiation factors. Potentiation factors that inhibit FUT8 include, but are not limited, to, RNAi agents that target FUT8 mRNA and/or antibodies and/or antigen-binding fragments of antibodies that bind to and inhibit FUT8 protein. RNAi agents include, but are not limited to, siRNAs, shRNAs, miRNAs, short antisense RNAs, and/or short dsRNAs.

Suitable RNAi agents (*e.g.,* siRNA, shRNA, miRNA, dsRNA, and antisense compounds) that inhibit expression of a target protein, such as FUT8 or other glycosylation enzyme, can be designed and synthesized using any suitable methods. Several methods for the design of such compounds are well-known in the art. See, *e.g*., Taxman, D.J. (Ed.), siRNA Design, Methods and Protocols, Human Press ISBN:978-1-62703-118-9 (2013); Naito et al., Nucleic Acids Research 32:W124-W129 (2004); Judge et al., Molecular Therapy 13:494-505 (2006); Lagana et al., Nucleic Acids Research doi: 10.1093/nar/gku202 (published on line March 13, 2014). Suitable methods and siRNA molecules for altering glycosylation are known in the art. See, for example, PCT patent application publications WO 2013/013013.

### 3. PHARMACEUTICAL COMPOSITIONS

The invention provides pharmaceutical compositions comprising exogenous nucleic acid molecules and a pharmaceutically acceptable carrier. If desired, other pharmaceutically acceptable components can be included, such as excipients and adjuvants. Pharmaceutical compositions optionally comprise a suitable nucleic acid delivery system as described in further detail below, such as a lipid nanoparticle, polymer nanoparticle, and/or oil-in-water emulsion. These compositions can be administered to a subject in need thereof to provide antibody expression and passive immunity.

Pharmaceutically acceptable carriers are determined in part by the particular composition being administered, as well as by the particular method used to administer the composition. Accordingly, there is a wide variety of suitable formulations of pharmaceutical compositions of the present invention. A variety of aqueous carriers can be used. Suitable pharmaceutically acceptable carriers for use in the pharmaceutical compositions include water (*e.g.* w.f.i.) or a buffer *e.g.* a phosphate buffer, a Tris buffer, a borate buffer, a succinate buffer, a histidine buffer, or a citrate buffer. Buffer salts will typically be included in the 5-20mM range.

Pharmaceutical compositions are preferably sterile, and may be sterilized by conventional sterilization techniques.

Pharmaceutical compositions may contain pharmaceutically acceptable auxiliary substances as required to approximate physiological conditions such as pH adjusting and buffering agents, and tonicity adjusting agents and the like, for example, sodium acetate, sodium chloride, potassium chloride, calcium chloride, sodium lactate and the like. Preferably, pharmaceutical compositions may have a pH between 5.0 and 9.5, e.g. between 6.0 and 8.0. If desired, pharmaceutical compositions can include one or more preservatives, such as thiomersal or 2-phenoxyethanol.

Pharmaceutical compositions of the invention are preferably non-pyrogenic e.g. containing <1 EU (endotoxin unit, a standard measure) per dose, and preferably <0.1 EU per dose. Pharmaceutical compositions of the invention are preferably gluten free.

The concentration of exogenous nucleic acid molecules in the pharmaceutical compositions can vary, and will be selected based on a number of factors, such as fluid volume for each dose, viscosities, body weight and other considerations in accordance with the particular mode of administration selected and the intended recipient's needs. However, pharmaceutical compositions are formulated to provide an effective amount of the exogenous nucleic acid molecule, such as an amount, either in a single dose or as part of a series, that is effective for providing expression of the antibody or antigen-binding fragment thereof in a subject in the desired degree. This amount varies depending upon the health and physical condition of the individual to be treated, age, the taxonomic group of individual to be treated (*e.g.* non-human primate, primate, *etc.*), the capacity of the individual to produce antibodies, the condition to be treated, and other relevant factors. It is expected that the amount will fall in a relatively broad range that can be determined through routine trials. It is expected that a dose will contain about 1ng to about 200 µg of exogenous nucleic acid molecules described herein.

The pharmaceutical compositions can be administered in any suitable way, such as parenterally, topically, by inhalation and the like. Pharmaceutical compositions suitable for parenteral administration (e.g., by intramuscular, intraperitoneal, subcutaneous or injection directly into a target organ (e.g., liver)) include aqueous and non-aqueous, isotonic sterile injection solutions, which can contain antioxidants, buffers, bacteriostats, and solutes that render the formulation isotonic with the blood of the intended recipient, and aqueous and non-aqueous sterile suspensions that can include suspending agents, solubilizers, thickening agents, stabilizers, and preservatives. Pharmaceutical compositions of exogenous nucleic acid molecules can be presented in unit-dose or multi-dose sealed containers, such as ampoules and vials. Injection solutions and suspensions can be prepared from sterile powders, granules, and tablets. Cells transfected by the exogenous nucleic acid molecules can also be administered intravenously or parenterally.

When a pharmaceutical composition is in the form of an emulsion, the exogenous nucleic acid molecules and emulsion can typically be mixed by simple shaking. Other techniques, such as passing a mixture of the emulsion and solution or suspension of the exogenous nucleic acid molecules rapidly through a small opening (such as a hypodermic needle), can be used to mix the pharmaceutical composition.

Pharmaceutical compositions suitable for oral administration can consist of (a) liquid solutions, such as an effective amount of the packaged nucleic acid suspended in diluents, such as water, saline or PEG 400; (b) capsules, sachets or tablets, each containing a predetermined amount of the active ingredient, as liquids, solids, granules or gelatin; (c) suspensions in an appropriate liquid; and (d) suitable emulsions. Tablet forms can include one or more of lactose, sucrose, mannitol, sorbitol, calcium phosphates, corn starch, potato starch, tragacanth, microcrystalline cellulose, acacia, gelatin, colloidal silicon dioxide, croscarmellose sodium, talc, magnesium stearate, stearic acid, and other excipients, colorants, fillers, binders, diluents, buffering agents, moistening agents, preservatives, flavoring agents, dyes, disintegrating agents, and pharmaceutically compatible carriers. Lozenge forms can comprise the active ingredient in a flavor, usually sucrose and acacia or tragacanth, as well as pastilles comprising the active ingredient in an inert base, such as gelatin and glycerin or sucrose and acacia emulsions, gels, and the like containing, in addition to the exogenous nucleic acid molecule, carriers known in the art. It is recognized that exogenous nucleic acid molecules, when administered orally, must be protected from digestion. This is typically accomplished either by complexing the exogenous nucleic acid molecules with a composition to render them resistant to acidic and enzymatic hydrolysis or by packaging the exogenous nucleic acid molecules in an appropriately resistant carrier such as a liposome. Means of protecting nucleic acids, such as exogenous nucleic acid molecules, from digestion are well known in the art. Pharmaceutical compositions can be encapsulated, e.g., in liposomes, or in a formulation that provides for slow release of the active ingredient.

Exogenous nucleic acid molecules, alone or in combination with other suitable components, can be made into aerosol formulations (*e.g*., they can be "nebulized") to be administered via inhalation. Aerosol formulations can be placed into pressurized acceptable propellants, such as dichlorodifluoromethane, propane, nitrogen, and the like.

### Nucleic Acid Delivery Systems

Exogenous nucleic acid molecules may be delivered to cells as a naked nucleic acid (*e.g.* merely as an aqueous solution of nucleic acid) or, optionally, with a nucleic acid delivery system. Suitable nucleic acid delivery systems include, but are not limited to, a lipid nanoparticle, a liposome, biodegradable polymer nanoparticle, and/or oil-in-water emulsion (including cationic emulsions). Such delivery systems are well known in the art and are described, for example, in co-pending U.S. patent application number 12/831,252, filed July 6, 2010, and published on December 8, 2011, as U.S. patent publication number 2011/0300205, the contents of which are incorporated herein by reference in its entirety.

For example, various lipids can form lipid bilayers to encapsulate the nucleic acid molecules described herein. These lipids can have an anionic, cationic or zwitterionic hydrophilic head group. Various polymers can form microparticles to encapsulate or adsorb the nucleic acid molecules described herein. Suitable nontoxic and biodegradable polymers include, but are not limited to, poly(α-hydroxy acids), polyhydroxy butyric acids, polylactones (including polycaprolactones), polydioxanones, polyvalerolactone, polyorthoesters, polyanhydrides, polycyanoacrylates, tyrosine-derived polycarbonates or polyester-amides, and combinations thereof.

Lipid nanoparticle (LNP) typically refers to a particle that comprises a plurality of (i.e. more than one) lipid molecules physically associated with each other by intermolecular forces. The lipid nanoparticles may be, e.g., microspheres (including unilamellar and multlamellar vesicles, e.g. liposomes), a dispersed phase in an emulsion, micelles or an internal phase in a suspension. A nucleic acid molecule may be encapsulated within an LPN or complexed with an LNP. It is not necessary that the lipid forms liposomes (with aqueous core) only. Some lipid nanoparticles may comprise a lipid core (e.g., the composition may comprise a mixture of liposomes and nanoparticles with a lipid core). In such cases, the nucleic acid molecules may be encapsulated by LNPs that have an aqueous core, and complexed with the LNPs that have a lipid core by non-covalent interactions (e.g., ionic interactions between negatively charged RNA and cationic lipid). Encapsulation and complexation with LNPs can protect RNA from RNase digestion. The encapsulation/complexation efficiency does not have to be 100%. Presence of "naked" RNA molecules (RNA molecules not associated with a liposome) is acceptable.

If desired, nucleic acid delivery systems may comprise one or more targeting moieties target the exogenous nucleic acid molecule to a particular organ, tissue, and/or cell type for transfection. For example, a nucleic acid delivery system may comprise a moiety that binds to a molecule (e.g., protein) found on the surface of target cell. Any suitable binding compound can be used as the binding moiety for targeting the delivery system to a target cell, such as, an antibody or antigen-binding fragment thereof, an aptamer, a nucleic acid, a carbohydrate, a receptor, a receptor ligand, and the like.

In some aspects, a nucleic acid delivery system may comprise a moiety (e.g., an antibody or antigen-binding fragment) that binds to a molecule (e.g., protein) found on the surface of the non-terminally differentiated B cells, including, but not limited to, IgD, CD34, CD45, CD19, CD20, CD38, CD40/TNFRSf5, CD84/SLAMF5, B220/CD45R, C1q R1/CD93, BAFF R/TNFRSF13C, CD24, CD1d, CD21, and/or combinations of the foregoing. Delivery of exogenous nucleic acid molecules to B cells may be achieved using any desired targeted delivery system, such as IgD targeted lipid nanoparticles.

In some aspects, a nucleic acid delivery system may comprise a moiety (e.g., an antibody or antigen-binding fragment) that binds to a molecule (e.g., protein) found on the surface of hepatocytes, including, but not limited to, cMET, ASGR1, ASGR2, SLC10A1, SLC38A4, LBP, SLC22A7, SLC38A3, TMPRSS6, SLC13A5, and/or combinations of the foregoing. Delivery of exogenous nucleic acid molecules to hepatocytes may be achieved using any desired targeted delivery system, such as cMET, ASGR1, ASGR2, SLC10A1, SLC38A4, LBP, SLC22A7, SLC38A3, TMPRSS6, and/or SLC13A5 targeted lipid nanoparticles.

In some aspects, a nucleic acid delivery system may comprise a moiety (e.g., an antibody or antigen-binding fragment) that binds to a molecule (e.g., protein) found on the surface of lung cells, including, but not limited to, SLC34A2, GPR116, AGER, and/or combinations of the foregoing. Delivery of exogenous nucleic acid molecules to lung cells may be achieved using any desired targeted delivery system, such as SLC34A2, GPR116, and/or AGER targeted lipid nanoparticles.

In some aspects, a nucleic acid delivery system may comprise a moiety (e.g., an antibody or antigen-binding fragment) that binds to a molecule (e.g., protein) found on the surface of Kupffer cells, muscle cells, skeletal cells, spleen cells, and/or plasma blasts.

### 4. METHODS OF ADMINISTRATION

Exogenous nucleic acid molecules (such as RNA) and/or pharmaceutical compositions described herein can be used to induce the production of the encoded antibody or antigen-binding fragment thereof in a subject, for example to achieve passive immunity for prophylactic or therapeutic purposes. Such methods generally involve locally or systemically administering an effective amount of exogenous nucleic acid molecule and/or pharmaceutical composition thereof to a subject in need thereof, whereby the exogenous nucleic acid molecule is transfected (e.g., transiently transfected) into the cells of the subject. The exogenous nucleic acid can be transfected into any desired cell such as liver cells (e.g., Kupffer cells and hepatocytes), muscle cells, skeletal cells, lung cells, spleen cells, immune system cells (e.g., mature plasmoblasts, B cells) and combinations of the foregoing. In preferred aspects, the exogenous nucleic acid can be transfected (transiently transfected) into a secretory cell or an immune system cell. The subject can be a vertebrate, such as a mammal (including, but not limited to, human, cattle, pig, horse, deer, sheep, goat, bison, rabbit, cat, dog, etc.), bird (including, but not limited to, chicken, duck, turkey, etc.), and/or fish.

Exogenous nucleic acid molecules and/or pharmaceutical compositions thereof can be used to treat patients susceptible to and/or suffering from infection by one or more pathogens (e.g., viruses, bacteria, fungi, protozoa, plants, and the like) by administering an effective amount of an exogenous nucleic acid molecule and/or pharmaceutical composition thereof that encodes an antibody and/or antigen-binding fragment of an antibody that binds to an antigen associated with the pathogen.

Exogenous nucleic acid molecules and/or pharmaceutical compositions thereof can be used to treat patients susceptible to and/or suffering from diseases in which diseased tissues or cells are characterized by expression of an alter endogenous antigen (e.g., a mutated protein) or altered expression of an endogenous antigen (e.g., increased or decreased expression of a protein) by administering an effective amount of an exogenous nucleic acid molecule and/or pharmaceutical composition thereof that encodes an antibody and/or antigen-binding fragment of an antibody that binds to the endogenous antigen. In one example, exogenous nucleic acid molecule and/or pharmaceutical compositions thereof can be used to treat patients susceptible to and/or suffering from cancer by administering an exogenous nucleic acid molecule and/or pharmaceutical composition thereof that results in expression of an antibody and/or antigen-binding fragment of an antibody that recognizes and specifically binds to an antigen associated with the cancer (e.g. a tumor antigen). While prophylactic or therapeutic treatment of the patient can be directed to any cancer or tumor cell, preferred cancers and tumor cells, include, but are not limited to, those described herein.

In preferred embodiments, exogenous nucleic acid molecules can be targeted to and transfected into cells that can produce and secrete high levels of antibody. Suitable cells of this type include cells associated with the immune system, such as B cells and plasma cells, and other types of secretory cells. Exogenous nucleic acid molecules may be delivered to undifferentiated B cells, substantially undifferentiated B cells, B cells at early stages of development, and/or B cells that have not been terminally differentiated, for example, using targeted delivery systems. Exogenous nucleic acid molecules may be targeted to and transfected into naive B cells. Without wishing to be bound by any particular theory, it is believed that naive B cells may have a flexible transcription program that can be rapidly and easily manipulated to induce terminal differentiation and/or maturation. Exogenous nucleic acid molecules may be delivered to naive IgD⁺ B cells using, for example, a targeted delivery system that includes a targeting moiety (e.g. and antibody or antigen-binding fragment thereof) that bind IgD. If desired, exogenous nucleic acid molecules may be delivered to liver cells, including, but not limited to, Kupffer cells and hepatocytes; muscle cells; skeletal cells; lung cells; spleen cells; mature plasmoblasts; and/or combinations of the foregoing.

Exogenous nucleic acid molecules may be delivered to cells located at or near the site where the exogenous nucleic acid molecules are administered, at or near the sight of injection. Exogenous nucleic acid molecules may be delivered to cells located at a site distant from the site where the exogenous nucleic acid molecules were administered to the subject.

Exogenous nucleic acid molecules, whether or not in association with a nucleic acid delivery system, may be administered using electroporation, injection and/or microinjection (e.g., intramuscular, intraperitoneal, intradermal, subcutaneous, intravenous, intraarterial, intraocular, intrathecal, intra-organ), a needleless injection device, lipofection, biolystics, and the like. Exogenous nucleic acid molecules, whether or not in association with a nucleic acid delivery system, may be administered topically or systemically. For example, topical administration can be achieved by intranasal administration, oromucosal administration, rectal administration, vaginal administration, ocular administration, contact with the skin, and the like. Exogenous nucleic acid molecules, whether or not in association with a nucleic acid delivery system, may be delivered to cells orally and/or via inhalation into the lung. Exogenous nucleic acid molecules, whether or not in association with a nucleic acid delivery system, may be delivered to a target organ and/or tissue via injection, a catheter or like device. Suitable catheters are disclosed in, *e.g.,* U.S. Pat. Nos. 4,186,745; 5,397,307; 5,547,472; 5,674,192; and 6,129,705 (each of which is incorporated herein by reference in its entirety).

Alternatively, cell-based therapy may be used. In a cell-based method, plasmablasts can be obtained from a subject. The plasmablasts are transfected in *vitro* with the nucleic acid molecule (such as RNA) described herein. Then, the plasmablasts are differentiated into mature antibody-secreting cells, which are then administered to a subject to confer immunity.

In general, an effective amount of an exogenous nucleic acid molecule and/or pharmaceutical composition thereof is administered to the patient. An effective amount of an exogenous nucleic acid molecule and/or pharmaceutical composition thereof may be administered to a subject in a single dose or as part of a series of doses. As described herein, this amount varies depending upon the health and physical condition of the subject to be treated, the condition to be treated, and other relevant factors.

An effective amount is generally an amount that is sufficient to achieve a desired level of expression of the antibody and/or antigen-binding fragment of an antibody. An effective amount can be an amount that is sufficient to achieve a high level of synthesis, high titer (e.g., serum concentration) of the antibody and/or antigen-binding fragment of an antibody. A level of synthesis of the antibody and/or antigen-binding fragment of an antibody can be at least 500 molecules/second, at least 1000 molecules /second, at least 1500 molecules /second, at least 2000 molecules /second, or at least 2500 molecules /second. A high titer of the antibody and/or antigen-binding fragment of an antibody can be a serum and/or plasma concentration of the antibody and/or antigen-binding fragment of an antibody of at least about 1 µg/L, at least about 2 µg/L, at least about 3 µg/L, at least about 4 µg/L, at least about 5 µg/L, and/or at least about 10 µg/L.

If desired, cells can be transfected with the exogenous nucleic acid molecule in vitro and the transfected cells can be administered to a subject in need thereof. For example, target cells may have been explanted from an individual (e.g., lymphocytes, bone marrow aspirates, tissue biopsy) and transfected in vitro. Transfect cells, with or without selection to enrich those cells that contain and express the exogenous nucleic molecule, can then be administered to a subject in need thereof. The appropriate amount of cells to deliver to a subject will vary with subject's conditions, desired effects and other factors and can be determined by a skilled artisan. See *e.g.,* U.S. Pat. Nos. 6,054,288; 6,048,524; and 6,048,729 (each of which is incorporated herein by reference). Preferably, the cells used are autologous, *i.e.,* cells obtained from the subject being treated.

Administration of an exogenous nucleic acid molecule and/or pharmaceutical composition thereof to a patient in need thereof may be performed concomitantly with other methods of achieving active or passive immunity, such as traditional vaccination procedures in which antigens (e.g., whole cells, viruses, purified antigens, immunogens, protein subunits, and/or peptide immunogenic compositions) are administered to a subject to trigger a protective and/or therapeutic immune response.

Exogenous nucleic acid molecules and/or pharmaceutical compositions thereof can be packaged in packs, dispenser devices, and/or kits. For example, packs or dispenser devices that contain one or more unit dosage forms are provided. Typically, instructions for administration will be provided with the packaging, along with a suitable indication on the label that the exogenous nucleic acid molecule and/or pharmaceutical composition thereof is suitable for treatment of an indicated condition. For example, the label may state that the exogenous nucleic acid molecule and/or pharmaceutical composition within the packaging is useful for achieving passive immunity to a particular pathogen and/or tumor.

### 5. DEFINITIONS

As used herein, a "cellular modulation factor" is factor, such as a protein or peptide, whose presence and/or activity results in increased expression of the antibody and/or antigen-binding fragment of an antibody relative to the expression obtained in the absence of the cellular modulation factor.

As used herein, an "exogenous" nucleic acid molecule is a nucleic acid molecule that is produced outside of a subject and introduced into a target cell found in a living subject so that it is present only for a transient time period. The exogenous nucleic acid molecule does not integrate into the genome of the target cell and is degraded or otherwise metabolized by the target cell or subject. The exogenous nucleic acid molecule may have the same nucleotide sequence found in nucleic molecules that are synthesized by the target cell (e.g., mRNA transcribed from the target cell genome).

As used herein, "expression" of an mRNA refers to translation of the protein which it encodes. "Expression" of an RNAi agent (e.g., siRNA, miRNA, antisense RNA, etc.) refers to completion of any requisite RNA processing events that must take place before the RNAi agent is available to perform its function (e.g., cleavage, formation of double- or single-stranded RNA, etc.).

As used herein, "passive immunity" refers to prophylactic and/or therapeutic immunity achieved in a subject by expression of protective antibodies and/or antigen-binding fragments of antibodies that are encoded by the exogenous nucleic acid molecule.

As used herein, a "potentiation factor" is a factor (e.g., protein, peptide, nucleic acid) whose presence and/or activity alters effector function and/or other properties of the expressed antibody or antigen-binding fragment (e.g., antibody dependent cell-mediated cytotoxicity, antibody dependent-cellular phagocytosis, complement fixation, immunogenicity). Potentiation factors include, for example, glycosylation enzymes and agents that inhibit glycosylation enzymes.

As used herein, "recombinant" refers to nucleic acids (e.g., DNA, RNA) that are produced by genetic manipulation in the laboratory to combine nucleic acids that were originally derived from one or more different sources. Recombinant nucleic acids may contain nucleic acids from the same species, nucleic acids from different species, and/or nucleic acids generated by chemical synthesis. Proteins that can result from the expression of recombinant DNA within living cells are generally referred to as "recombinant proteins." Recombinant nucleic acids and recombinant proteins are not generally found in nature.

### 6. EXEMPLARY EMBODIMENTS

The subject technology is illustrated, for example, according to various aspects described below. Various examples of aspects of the subject technology are described as numbered embodiments (1, 2, 3, etc.) for convenience. These are provided as examples and do not limit the subject technology. It is noted that any of the dependent clauses may be combined in any combination, and placed into a respective independent clause, e.g., embodiment 1 or embodiment 2. The other embodiment can be presented in a similar manner.
1. A recombinant RNA molecule comprising:
   an RNA sequence encoding an antibody, or antigen-binding fragment of an antibody, that is operably linked to one or more expression control elements, thereby in a suitable host cell resulting in the production of said antibody or antigen-binding fragment.
2. An *in vitro* transcribed recombinant RNA molecule comprising:
   an RNA sequence encoding an antibody, or antigen-binding fragment of an antibody, that is operably linked to one or more expression control elements, thereby in a suitable host cell resulting in the production of said antibody or antigen-binding fragment.
3. The recombinant RNA molecule of embodiment 1 or 2, wherein said antibody, or antigen-binding fragment thereof, comprises: a heavy chain or the V_{H} domain of the heavy chain, and a light chain or the V_{L} domain of the light chain.
4. The recombinant RNA molecule of embodiment 3, wherein the coding sequence for said heavy chain or V_{H} domain, and the coding sequence for said light chain or V_{L} domain are in a single open reading frame.
5. The recombinant RNA molecule of embodiment 3 or 4, wherein the RNA molecule comprises one or more expression control elements that is located between the coding sequence for said heavy chain or V_{H} domain, and the coding sequence for said light chain or V_{L} domain.
6. The recombinant RNA molecule of embodiment 5, wherein the one or more control elements are independently selected from the group consisting of a subgenomic promoter, an IRES, a viral 2A site.
7. The recombinant RNA molecule of any one of embodiments 3-6, wherein said heavy chain or V_{H} domain, and said light chain or V_{L} domain are covalently linked by a linker.
8. The recombinant RNA molecule of embodiment 7, wherein said linker is a cleavable linker.
9. The recombinant RNA molecule of any one of embodiments 1-8, wherein said RNA comprises one or more modified nucleotides.
10. The recombinant RNA molecule of any one of embodiments 1-9, wherein said RNA is an mRNA construct.
11. The recombinant RNA molecule of any one of embodiments 1-9, wherein said RNA is a self-replicating RNA.
12. The recombinant RNA molecule of embodiment 11, wherein said self-replicating RNA is an alphavirus replicon.
13. The recombinant RNA molecule of any one of embodiments 1-12, wherein the host cell is a mammalian cell.
14. The recombinant RNA molecule of any one of embodiments 1-13, wherein the host cell is a human cell.
15. The recombinant RNA molecule of any one of embodiments 1-14, wherein said host cell is a plasmablast cell.
16. The recombinant RNA molecule of any one of embodiments 1-14, wherein said host cell is a naive B cell.
17. The recombinant RNA molecule of any one of embodiments 1-14, wherein the host cell is selected from the group consisting of liver cell, muscle cell, skeletal cell, lung cell, spleen cell and combinations thereof.
18. The recombinant RNA molecule of any one of embodiments 1-17, wherein said RNA molecule further comprises: (i) an RNA sequence encoding a cellular modulation factor, (ii) an RNA sequence encoding a potentiation factor, or (iii) a combination thereof.
19. The recombinant RNA molecule of embodiment 18, wherein the cellular modulation factor is selected from the group consisting of: a growth factor, a cytokine, a transcription factor, a chaperone protein, a translocation protein, a processing protein, a transporter protein, and a combination thereof.
20. The recombinant RNA molecule of embodiment 18 or 19, wherein the cellular modulation factor causes differentiation of the suitable cell, and/or causes expansion of the endoplasmic reticulum and/or Golgi.
21. The recombinant RNA molecule of any one of embodiments 18-20, wherein the cellular modulation factor is STXBP3.
22. The recombinant RNA molecule of embodiment 18, wherein the potentiation factor is a glycosylation control agent.
23. The recombinant RNA molecule of embodiment 22, wherein the glycosylation control agent inhibits a glycosylation enzyme.
24. The recombinant RNA molecule of embodiment 22 or 23, wherein the glycosylation control agent is an inhibitor of a fucosyltransferase enzyme.
25. The recombinant RNA molecule of any one of embodiments 22-24, wherein the glycosylation control agent is an inhibitor of FUT8.
26. The recombinant RNA molecule of any one of embodiments 22-25, wherein the glycosylation control agent is an RNAi agent or miRNA.
27. The recombinant RNA molecule of embodiment 22, wherein the glycosylation control agent is a glycosylation enzyme.
28. The recombinant RNA molecule of embodiment 22 or 27, wherein the glycosylation control agent is MGAT3.
29. The recombinant RNA molecule of any one of embodiments 18-28, wherein said RNA sequence encoding a cellular modulation factor, said RNA sequence encoding a potentiation factor, or both, are operably linked to one or more expression control elements.
30. The recombinant RNA molecule of embodiment 29, wherein the one or more control elements are independently selected from the group consisting of a subgenomic promoter, an IRES, a viral 2A site.
31. The recombinant RNA molecule of any one of embodiments 1-30, wherein the antibody or antigen-binding fragment has binding specificity for a pathogen antigen or an endogenous antigen.
32. The recombinant RNA molecule of any one of embodiments 1-31, wherein the antibody or antigen-binding fragment is produced in the host cell at a rate of at least about 500 molecule/second.
33. A composition comprising the recombinant RNA molecule of any one of embodiments 1-32, and a delivery system suitable for delivering the RNA into a suitable host cell.
34. The composition of embodiment 33, wherein the system is selected from the group consisting of a lipid nanoparticle, a polymer nanoparticle, a liposome, and an oil-in-water emulsion.
35. The composition of embodiment 34, wherein the delivery system targets a B cell.
36. The composition of embodiment 34, wherein said composition comprises an agent that binds to IgG.
37. The composition of embodiment 34, wherein the delivery system targets a plasmablast.
38. A B cell comprising the recombinant RNA molecule of any one of embodiments 1-32.
39. The B cell of embodiment 38, wherein said B cell is a naive B cell.
40. A plasmablast comprising the recombinant RNA molecule of any one of embodiments 1-32.
41. The plasmablast of embodiment 40, wherein said plasmablast is CD19+ CD20-/lo.
42. The recombinant RNA molecule of any one of embodiments 1-32, or the composition of any one of embodiments 34-37, the B cell of embodiment 38 or 39, or the plasmablast of embodiment 40 or 41, for use in producing an antibody in a subject.
43. A method of producing an antibody *in vivo,* comprising administering to a subject in need thereof, a therapeutically effective amount of the recombinant RNA molecule of any one of embodiments 1-32, or the composition of any one of embodiments 34-37, the B cell of embodiment 38 or 39, or the plasmablast of embodiment 40 or 41.
44. The method of embodiment 43, wherein said administration is by electroporation, injection, inhalation or intra-nasal delivery.
45. The method of embodiment 44, wherein said injection is selected from the group consisting of intramuscular, intravenous, intraarterial, subcutaneous, intrathecal, intradermal, intraperitoneal, intra-organ, and intraocular injection.
46. The method of any one of embodiments 43-45, wherein said administration results in a serum or plasma concentration of antibody or antigen-binding fragment of at least about 1 microgram/L.
47. The method of one of embodiments 43-46, wherein said administration results in a peak plasma level of said antibody in said subject in a range from about 3 µg/ml to about 30 µg/ml.
48. A method of producing an antibody *in vivo* in a subject in need thereof, comprising
   (1) providing a population of plasmablasts from said subject;
   (2) introducing the recombinant RNA molecule of any one of embodiments 1-32 into said plasmablasts in *vitro*;
   (3) inducing differentiation of said plasmablasts into mature antibody-secreting cells; and
   (4) administering said antibody-secreting cells into said subject.
49. A recombinant nucleic acid molecule comprising:
   a first polynucleotide sequence encoding an antibody, or antigen-binding fragment of an antibody, that is operably linked to one or more expression control elements,
   thereby in a suitable host cell resulting in the production of said antibody or antigen-binding fragment,
   wherein said polynucleotide sequence does not comprise an intron.
50. The recombinant nucleic acid molecule of embodiment 49, further comprises: (i) a second polynucleotide sequence encoding a cellular modulation factor, (ii) a third polynucleotide sequence encoding a potentiation factor, or (iii) a combination thereof.
51. The recombinant nucleic acid molecule of embodiment 49 or 50, wherein said first polynucleotide sequence, second polynucleotide sequence, if present, and third polynucleotide sequence, if present, are cDNA sequences.
52. The recombinant nucleic acid molecule of any one of embodiments 49-51, wherein said antibody, or antigen-binding fragment thereof, comprises: a heavy chain or the V_{H} domain of the heavy chain, and a light chain or the V_{L} domain of the light chain.
53. The recombinant nucleic acid molecule of any one of embodiments 49-52, wherein the coding sequence for said heavy chain or V_{H} domain, and the coding sequence for said light chain or V_{L} domain are in a single open reading frame.
54. The recombinant nucleic acid molecule of embodiment 52 or 53, comprising one or more expression control elements located between the coding sequence for said heavy chain or V_{H} domain, and the coding sequence for said light chain or V_{L} domain.
55. The recombinant nucleic acid molecule of embodiment 54, wherein said one or more control elements are independently selected from the group consisting of a subgenomic promoter, an IRES, a viral 2A site.
56. The recombinant nucleic acid molecule of any one of embodiments 52-55, wherein said heavy chain or V_{H} domain, and said light chain or V_{L} domain are covalently linked by a linker.
57. The recombinant nucleic acid molecule of embodiment 56, wherein said linker is a cleavable linker.
58. The recombinant nucleic acid molecule of any one of embodiments 49-57, comprising one or more modified nucleotides.
59. The recombinant nucleic acid molecule of any one of embodiments 49-58, wherein the host cell is a mammalian cell.
60. The recombinant nucleic acid molecule of any one of embodiments 49-59, wherein the host cell is a human cell.
61. The recombinant nucleic acid molecule of any one of embodiments 49-60, wherein said host cell is a plasmablast cell.
62. The recombinant nucleic acid molecule of any one of embodiments 49-61, wherein said host cell is a naive B cell.
63. The recombinant nucleic acid molecule of any one of embodiments 49-62, wherein the host cell is selected from the group consisting of liver cell, muscle cell, skeletal cell, lung cell, spleen cell and combinations thereof.
64. The recombinant nucleic acid molecule of any one of embodiments 50-63, wherein the cellular modulation factor is selected from the group consisting of: a growth factor, a cytokine, a transcription factor, a chaperone protein, a translocation protein, a processing protein, a transporter protein, and a combination thereof.
65. The recombinant nucleic acid molecule of embodiment 64, wherein the cellular modulation factor causes differentiation of the suitable cell, and/or causes expansion of the endoplasmic reticulum and/or Golgi.
66. The recombinant nucleic acid molecule of embodiment 64 or 65, wherein the cellular modulation factor is STXBP3.
67. The recombinant nucleic acid molecule of any one of embodiments 50-66, wherein the potentiation factor is a glycosylation control agent.
68. The recombinant nucleic acid molecule of embodiment 67, wherein the glycosylation control agent inhibits a glycosylation enzyme.
69. The recombinant nucleic acid molecule of embodiment 67 or 68, wherein the glycosylation control agent is an inhibitor of a fucosyltransferase enzyme.
70. The recombinant nucleic acid molecule of any one of embodiments 67-69, wherein the glycosylation control agent is an inhibitor of FUT8.
71. The recombinant nucleic acid molecule of any one of embodiments 67-70, wherein the glycosylation control agent is an RNAi agent or miRNA.
72. The recombinant nucleic acid molecule of embodiment 67, wherein the glycosylation control agent is a glycosylation enzyme.
73. The recombinant nucleic acid molecule of embodiment 67 or 72, wherein the glycosylation control agent is MGAT3.
74. The recombinant nucleic acid molecule of any one of embodiments 50-73, wherein said polynucleotide sequence encoding a cellular modulation factor, said polynucleotide sequence encoding a potentiation factor, or both, are operably linked to one or more expression control elements.
75. The recombinant nucleic acid molecule of embodiment 74, wherein the one or more control elements are independently selected from the group consisting of a subgenomic promoter, an IRES, a viral 2A site.
76. The recombinant nucleic acid molecule of any one of embodiments 49-75, wherein the antibody or antigen-binding fragment has binding specificity for a pathogen antigen or an endogenous antigen.
77. The recombinant nucleic acid molecule of any one of embodiments 49-76, wherein the antibody or antigen-binding fragment is produced in the host cell at a rate of at least about 500 molecule/second.
78. A composition comprising the recombinant nucleic acid molecule of any one of embodiments 49-77, and a delivery system suitable for delivering the nucleic acid molecule into a suitable host cell.
79. The composition of embodiment 78, wherein the system is selected from the group consisting of a lipid nanoparticle, a polymer nanoparticle, a liposome, and an oil-in-water emulsion.
80. The composition of embodiment 78, wherein the delivery system targets a B cell.
81. The composition of embodiment 78, wherein said composition comprises an agent that binds to IgG.
82. The composition of embodiment 78, wherein the delivery system targets a plasmablast.
83. A B cell comprising the recombinant nucleic acid molecule of any one of embodiments 49-77.
84. The B cell of embodiment 83, wherein said B cell is a naive B cell.
85. A plasmablast comprising the recombinant nucleic aicd molecule of any one of embodiments 49-77.
86. The plasmablast of embodiment 85, wherein said plasmablast is CD19+ CD20-/lo.
87. The recombinant nucleic acid molecule of any one of embodiments 49-77, or the composition of any one of embodiments 78-82, the B cell of embodiment 83 or 84, or the plasmablast of embodiment 85 or 86, for use in producing an antibody in a subject.
88. A method of producing an antibody *in vivo,* comprising administering to a subject in need thereof, a therapeutically effective amount of the recombinant nucleic acid molecule of any one of embodiments 49-77, or the composition of any one of embodiments 78-82, the B cell of embodiment 83 or 84, or the plasmablast of embodiment 85 or 86.
89. The method of embodiment 88, wherein said administration is by electroporation, injection, inhalation or intra-nasal delivery.
90. The method of embodiment 89, wherein said injection is selected from the group consisting of intramuscular, intravenous, intraarterial, subcutaneous, intrathecal, intradermal, intraperitoneal, intra-organ, and intraocular injection.
91. The method of any one of embodiments 88-90, wherein said administration results in a serum or plasma concentration of antibody or antigen-binding fragment of at least about 1 microgram/L.
92. The method of one of embodiments 88-91, wherein said administration results in a peak plasma level of said antibody in said subject in a range from about 3 µg/ml to about 30 µg/ml.
93. A method of producing an antibody *in vivo* in a subject in need thereof, comprising
   (1) providing a population of plasmablasts from said subject;
   (2) introducing the recombinant nucleic acid molecule of any one of embodiments 49-77 into said plasmablasts in *vitro*;
   (3) inducing differentiation of said plasmablasts into mature antibody-secreting cells; and
   (4) administering said antibody-secreting cells into said subject.
94. An isolated cell comprising a recombinant RNA molecule of any one of embodiments 1-33 or a recombinant nucleic acid molecule of any one of embodiments 49-77.
95. The recombinant RNA molecule of any one of the preceding embodiments, the composition comprising said recombinant RNA molecule and a delivery system suitable for delivering said RNA into a suitable host cell, or a B cell or plasmablast comprising said recombinant RNA molecule, for use in a method of producing an antibody *in vivo* in a subject in need thereof..
96 A method of producing antibody-secreting cells comprising:
   (1) providing a population of plasmablasts from a subject;
   (2) introducing the recombinant RNA molecule of any one of the preceding embodiments into said plasmablasts in *vitro,* and
   (3) inducing differentiation of said plasmablasts into mature antibody-secreting cells, preferably in *vitro.*
97. Mature antibody-secreting cells obtainable by the method of embodiment 96 for use in a method of producing an antibody *in vivo* in a subject in need thereof.

### EXAMPLES

### Example 1. Expression of mRNA and Replicon RNA in a Variety of Cell Types

### A. mRNA and replicon RNA are expressed in muscle cells.

The ability of replicon RNA (replicon) and mRNA to induce gene expression in the muscle cell line, C2C12, was analyzed by fluorescence microscopy following transfection of an mRNA or replicon encoding mVenus. The replicon consisted of a Venezuelan Equine Encephalitis virus backbone in which the structural proteins were replaced with a construct encoding mVenus. The mRNA consisted of the mVenus cDNA preceded by the Venezuelan Equine Encephalitis virus 5' UTR and followed by the Venezuelan Equine Encephalitis virus 3' UTR. Both the mRNA and replicon RNA were synthesized, capped, and polyadenylated *in vitro.* C2C12 cells (10⁵) were transfected via electroporation, plated in 24-well plates, and imaged 24, 48, 96, and 120 hours post-transfection. Baseline fluorescence was established on electroporated C2C12 cells that did not receive RNA. Peak expression following mRNA transfection was observed at 24 hours. In contrast, replicon mVenus expression continued to increase over time, peaking at 96 hrs post-transfection. Significant differences were observed both in the kinetics of mVenus expression and in peak expression among the two RNA-based delivery/expression approaches, as determined by fluorescence assays.

### B. Enhanced antibody expression from replicon RNA

Differences in the ability of mRNA and replicon to induce antibody secretion from C2C12 cells *in vitro* were compared. C2C12 cells (10⁵) were transfected with an mRNA or replicon encoding the anti-influenza antibody F10. The replicon consisted of a Venezuelan Equine Encephalitis virus backbone in which the structural proteins were replaced with a construct encoding the heavy and light chains of the anti-influenza antibody F10 separated by a picornaviral 2A sequence. The mRNA consisted of the F10 heavy and light chain sequences separated by a picornaviral 2A sequence preceded by the Venezuelan Equine Encephalitis virus 5' UTR and followed by the Venezuelan Equine Encephalitis virus 3' UTR. Both the mRNA and replicon RNA were synthesized, capped, and polyadenylated in *vitro.* Supernatants were then collected at 2, 4, 8, and 24 hours post-transfection. Similar to mVenus expression, rapid production of antibody *in vitro* was observed by both mRNA- and replicon-based gene delivery. Replicon-based antibody gene transfer led to more robust antibody expression levels at every time point tested. Additionally, while mRNA technology achieved peak antibody expression levels 4 hours post-transfection, replicon-based antibody expression continued to increase over the course of the first 24 hours (Figure 1).

### C. mRNA and Replicon express in diverse immunocompetent cells

Because replicons replicate so rapidly, this gene transfer approach may trigger innate immune sensors, resulting in limited potential expression in immunocompetent cells. Thus, to define the landscape of potential cell types that may be targetable by replicon for antibody expression, a panel of immunoreplete cell lines were probed for mVenus expression. mVenus was detectable in all probed cell types, suggesting that the replicon can replicate effectively in many different cell types. Although lower transfection efficiencies in Jeko-1 (B cells) led to lower expression levels, robust replicon expression was observed in this cell type on a per-cell level.

### D. Replicon expression persists in vitro for long periods of time

Given the rapid and robust expression of genes from the replicon, as discussed above, the level of persistent replicon-mediated gene expression was examined in C2C12 and Hepa1-6 cells over the course of 1 month. Replicon expression peaked over the first week of culture, but replicon expression continued high levels over a month in both muscle C2C12 and Hepa1-6 cells.

### Example 2. Antibody Expression In Vivo

Next, the capacity of replicon-mediated gene transfer to drive antibody production *in vivo* was assessed. Mice were injected intra-muscularly with lipid-nanoparticles containing a replicon encoding both luciferase (Luc) and an anti-influenza antibody (Fi6v3). The replicon comprises a Venezuelan Equine Encephalitis virus backbone in which the structural proteins were replaced with a bicistronic construct encoding (1) the heavy and light chains of the anti-influenza antibody F10 separated by a picornaviral 2A sequence and (2) firefly luciferase. The two cistrons were connected via an internal ribosome entry site. The bicistronic mRNA encoded (1) the F10 heavy and light chain sequences separated by a picornaviral 2A sequence and (2) firefly luciferase. The two cistrons were connected via an internal ribosome entry site and together were preceded by the Venezuelan Equine Encephalitis virus 5' UTR and followed by the Venezuelan Equine Encephalitis virus 3' UTR. Both the mRNA and replicon RNA were synthesized, capped, and polyadenylated *in vitro.* The RNA was formulated with lipid-nanoparticles comprising DOTAP. In general, the DOTAP-LNP comprises about 40% DOTAP, about 10% DPE, about 48% cholesterol and about 2% PEG-DMG. For comparison, naked plasmid DNA (20 µg) was also used. The replicon induced robust local expression of luciferase. Similarly, rapid production of detectable levels of anti-influenza human monoclonal antibodies was observed in mouse serum.

### A. Robust and reproducible production of human monoclonals following replicon-mediated gene transfer in vivo

The reproducible production of monoclonal antibodies was tested in a cohort of eight mice inoculated with nanoparticles delivering 50 µg of anti-influenza F10 antibody-encoding replicon intravenously. All mice showed rapid peripheral antibody levels in the 50 ng/ml range, equivalent to approximately 3 µg/kg of antibody. Two mice reached approximately 200 ng/ml of antibody within 3 days of inoculation, with one mouse increasing steadily thereafter to 250 ng/ml levels. These data suggest that superior antibody levels may be reached using replicon-mediated gene transfer that may be related to differential replicon restriction and/or differential replicon delivery to target cells, both of which can be optimized.

### Example 3. Co-Delivery of Cellular Modulation Factors and/or

### Potentiation Factors

### A. Increased expression by co-delivery of cellular modulation factors

Limitations in antibody secretion levels *in vitro* and *in vivo* may be related to several factors including: a) poor replicon delivery *in vivo,* b) replicon restriction following innate immune sensing in cells, and/or c) limitations in the protein production capacity of the transfected cells. To overcome the latter, it is possible that replicon gene expression may be enhanced via the co-delivery of genes that may increase the secretory capacity of the transfected cells (cellular modulation factors). To this end a number of genes involved in protein secretion, grouped into genes involved in exocytosis (SCFD, STXBP1, STXBP2, and/or SYXBOP3), folding (PDIA2, PPIB, HSPA5, and/or MZB1), translocation (SEC61A1, SEC61A2, SEC61B, and/or SEC61G), and signal peptide recognition (SRPR, SRPRB, SRP14, SRP54, and/or SRP9) were transfected into 293T cells, C2C12 (muscle) cells, and Hepa1-6 (hepatocyte) cells, and antibody production was analyzed. Several genes increased antibody expression above levels observed at baseline in each of the cell types, including several exocytosis factors, translocation, and transcription factors. Over-expression of STXBP3 resulted in more than a 600% increase in antibody production in muscle cells. Moreover, increased antibody expression following gene-overexpression was linked to increased ER-size. Results are summarized in Table 2:

**Table 2: Increased Antibody Expression by Co-Delivery of Cellular Modulation Factors**

| Pathway | Gene | Percent of Control Expression | |
|---|---|---|---|
| | | Muscle | Liver |
| Exocytic Vesicle Trafficking | SCFD | 106% | 178% |
| | STXBP1 | 259% | 235% |
| | STXBP2 | 169% | 89% |
| | STXBP3 | 625% | 315% |
| Chaperones | PDIA2 | 25% | 86% |
| | PPIB | 25% | 87% |
| | HSPA5 | 25% | 77% |
| | MZB1 | 26% | 81% |
| ER Translocation | SEC61A1 | 17% | 78% |
| | SEC61A2 | 34% | 84% |
| | SEC61B | 32% | 116% |
| | SEC61G | 64% | 215% |
| Signal Petide Recognition | SRPRB | 15% | 88% |
| | SRP14 | 130% | 90% |
| | SRP54 | 128% | 128% |
| | SRP9 | 130% | 86% |
| | SRPR | 128% | 124% |
| Transcription Factors | ATF6C | 102% | 177% |

### B. Co-delivery of Potentiation Factors to improve antibody Fc-function

In addition to augmenting antibody levels *in vivo,* the optimized delivery of "functional" antibodies could augment the clinical efficacy of the monoclonal antibodies delivered via RNA based delivery strategies. Significant effort in the monoclonal therapeutics field has been invested in the development of antibody producer cell lines that generate glycan-optimized antibodies including afucosylated variants that exhibit enhanced ADCC and afucosylated/bisected variants that exhibit enhanced ADCC and complement activity. Here, accessory gene modifiers were co-delivered with antibody genes that can ensure the *in vivo* production of similar and potentially a broader repertoire of antibody modifications.

Two approaches were used: 1) for the knock down of glycosyltransferases (required to generate afucosylated antibodies), siRNAs that effectively knock down FUT8 (the fucosyltransferase involved in adding the alpha1-6 fucose to the antibody glycan) were adapted into a miRNA that can be processed in cells into an siRNA that can knock down the gene, and 2) additional glycosyltransferases were overexpressed to add other carbohydrates (such as the bisecting GlcNAc by MGAT3). Additional glycosyltransferases and/or glycosidases can be silenced or over-expressed, depending on the target glycan structure and the expression pattern of these enzymes in the particular cell, to tune the glycan to gain optimal function. Silencing of FUT8 expression was more effective when the FUT8-specific siRNA was delivered via the replicon compared with the traditional delivery of the siRNA. Moreover, the replicon-mediated delivery of the FUT8-specific siRNA demonstrated persistent silencing, suggesting that simultaneous, replicon-mediated delivery of antibody genes with miRNAs targeting specific glycosyltransferases will allow for the rapid production of highly functional, monoclonal antibodies with tuned effector functions *in vivo.*

### Example 4. Delivery of exogenous nucleic acid molecule to B cells

Beyond the in cell improvement of both antibody production and function, targeting of the replicon to optimized cells of the immune system may represent an alternative approach for the production of large quantities of highly functional antibodies. Specifically, antibody secreting cells (ASCs) secrete over 1500 antibodies per second and generate glycans optimized for function. Thus, the present inventors recognized that the targeted delivery of mRNA or replicon to naive or memory B cells that can then be induced to differentiate into ASCs or into mature non-IgG B cells (to avoid immunoglobulin rearrangement and reduced clinical efficacy) may boost functional antibody production. Indeed, delivery of antibody encoding replicons to mature non-IgG+ B cells followed by maturation to an ASC results in a 80-fold increase in antibody production levels, and ASCs can be readily transfected with replicons (Figure 2). Thus, the delivery of antibody-encoding mRNA or replicon to cells naturally optimized for antibody production, our bodies "antibody-factories," provides an alternate rapid mechanism by which to induce clinically efficacious doses of Fc-enhanced antibodies *in vivo.*

Throughout this application, various publications have been referenced. The disclosures of these publications in their entireties are hereby incorporated by reference.

Although the invention has been described with reference to the disclosed embodiments, those skilled in the art will readily appreciate that the specific embodiments, examples, and studies detailed herein are only illustrative of the invention. It should be understood that various modifications can be made without departing from the spirit of the invention.

The various features and embodiments of the present invention, referred to in individual sections above apply, as appropriate, to other sections, mutatis mutandis. Consequently features specified in one section may be combined with features specified in other sections, as appropriate.

Those skilled in the art will recognize, or be able to ascertain using no more than routine experimentation, many equivalents to the specific embodiments of the invention described herein. Such equivalents are intended to be encompassed by the following claims.

## Claims

1. A recombinant RNA molecule comprising:
an RNA sequence encoding an antibody, or antigen-binding fragment of an antibody, that is operably linked to one or more expression control elements, thereby in a suitable host cell resulting in the production of said antibody or antigen-binding fragment.

2. The recombinant RNA molecule of claim 1, wherein said antibody, or antigen-binding fragment thereof, comprises: a heavy chain or the V_{H} domain of the heavy chain, and a light chain or the V_{L} domain of the light chain.

3. The recombinant RNA molecule of claim 2, wherein the coding sequence for said heavy chain or V_{H} domain, and the coding sequence for said light chain or V_{L} domain are in a single open reading frame.

4. The recombinant RNA molecule of claim 2 or 3, wherein the RNA molecule comprises one or more expression control elements that is located between the coding sequence for said heavy chain or V_{H} domain, and the coding sequence for said light chain or V_{L} domain.

5. The recombinant RNA molecule of any one of claims 2-4, wherein said heavy chain or V_{H} domain, and said light chain or V_{L} domain are covalently linked by a linker

6. The recombinant RNA molecule of any one of claims 1-5, wherein said RNA is an mRNA construct.

7. The recombinant RNA molecule of any one of claims 1-5, wherein said RNA is a self-replicating RNA.

8. The recombinant RNA molecule of any one of claims 1-7, wherein said RNA molecule further comprises: (i) an RNA sequence encoding a cellular modulation factor, (ii) an RNA sequence encoding a potentiation factor, or (iii) a combination thereof.

9. The recombinant RNA molecule of claim 8, wherein the cellular modulation factor is selected from the group consisting of: a growth factor, a cytokine, a transcription factor, a chaperone protein, a translocation protein, a processing protein, a transporter protein, and a combination thereof.

10. The recombinant RNA molecule of claim 8 or 9, wherein the cellular modulation factor causes differentiation of the suitable cell, and/or causes expansion of the endoplasmic reticulum and/or Golgi.

11. The recombinant RNA molecule of claim 8, wherein the potentiation factor is a glycosylation control agent.

12. A composition comprising the recombinant RNA molecule of any one of claims 1-11, and a delivery system suitable for delivering the RNA into a suitable host cell.

13. A B cell or plasmablast comprising the recombinant RNA molecule of any one of claims 1-11.

14. A method of producing an antibody *in vivo,* comprising administering to a subject in need thereof, a therapeutically effective amount of the recombinant RNA molecule of any one of claims 1-11, or the composition of claim 12, or the B cell or the plasmablast of claim 13.

15. A method of producing an antibody *in vivo* in a subject in need thereof, comprising:
(1) providing a population of plasmablasts from said subject;
(2) introducing the recombinant RNA molecule of any one of claims 1-11 into said plasmablasts *in vitro*;
(3) inducing differentiation of said plasmablasts into mature antibody-secreting cells; and
(4) administering said antibody-secreting cells into said subject.
